# EUROPEAN PATENT SPECIFICATION

(11) **EP 0 413 516 B1**
(45) Date of publication and mention of the grant of the patent: **20.04.1994**
(21) Application number: 90308783.1
(22) Date of filing: 09.08.1990
(51) Int. Cl.: C07D 491/10, C07D 498/10, A61K 31/42, A61K 31/445

(54) **4-Methylene-1-oxa-2-oxo-8-azaspiro[4,5]decane derivatives, pharmaceutical compositions containing them and processes for preparing them**
4-Methylen-1-oxa-2-oxo-8-azaspiro[4,5]decanderivate, Verfahren zu ihrer Herstellung und diese enthaltende pharmazeutische Zubereitungen
Dérivés de 4-méthylène-1-oxa-2-oxo-8-azaspiro[4,5]decane, procédé pour leur préparation et compositions pharmaceutiques les contenant

(30) Priority: 10.08.1989 HU 409489
(43) Date of publication of application: 20.02.1991
(73) Proprietor: RICHTER GEDEON VEGYESZETI GYAR R.T., H-1475 Budapest (HU)
(72) Inventor: Toth, Edit, H-1114 Budapest (HU); Törley, Jozsef, H-1137 Budapest (HU); Görög, Sandor, Dr., H-1103 Budapest (HU); Szporny, Laszlo, Dr., H-1114 Budapest (HU); Kiss, Bela, H-1103 Budapest (HU); Palosi, Eva, Dr., H-1025 Budapest (HU); Groo, Dora, Dr., H-1021 Budapest (HU); Laszlovszky, Istvan, Dr., H-1111 Budapest (HU); Lapis, Erzsebet, Dr., H-1173 Budapest (HU); Auth, Ferenc, H-1024 Budapest (HU); Gaal, Laszlo, Dr., H-1103 Budapest (HU)
(74) Representative: Pett, Christopher Phineas

(56) References cited:
- FR-A- 2 262 666
- GB-A- 1 348 089
- JOURNAL OF MEDICINAL CHEMISTRY, vol. 10, no. 4, 1967, pages 627-635, Washington DC, US; M.A. DAVIS et al.: "New psychotropic agents. VIII. Analogs of amitriptyline containing the normeperidine group"
- CHIMIE THERAPEUTIQUE, vol. 7, no. 6, 1972, pages 458-466, Paris, FR; J. MAILLARD et al.: "Composés cycloalcanespirohétérocycliques VIII. Dérivés de (pipéridine-4' spiro) 5 oxazolidinones-2 (oxa-1 diaza-3,8 spiro [4,5] décanones-2)"
- CHEMICAL ABSTRACTS, vol. 80, no. 15, 15th April 1974, pages 366-367, abstract no. 82582e, Columbus, Ohio, US; K. SINDELAR et al.: "Neurotropic and psychotropic agents. LXVII. 1-[4,4-Bis(4-fluorophenyl)butyl]-4-hydroxy-4-[3-(trifluoromethyl)-4-chlorophenyl]piperidine and related compounds. New synthetic approaches"

## Description

This invention relates to 4-methylene-1-oxa-2-oxo-8-azaspiro[4,5]decane derivatives, pharmaceutical compounds containing them and processes for preparing them.

A number of therapeutically useful 2-oxo-1-oxa-3,8-di-azaspiro[4,5]decane derivatives have been described in the literature, e.g. C.A. 71, 91359d (1969); C.A. 78, 23876q (1973); C.A. 80, 82582e (1974); C.A. 81, 33153c and 105368b (1974); C.A. 95, 161765e (1981); Chimie Therapeutique, 7 (1972) pp 458-466; as well as in the DE patent specifications Nos. 2,013,729, 2,013,668 and 2,163,000 and in the BE patent specifications Nos. 775,984, 774,170, 786,631 and 825,444; in the GB patent specification No. 1,100,281; in the published NL patent specification No. 7,214,689; as well as in the US patent specifications Nos. 3,555,033, 3,594,386, 4,244,961 and 4,255,432.

We have now developed a novel range of compounds wherein the nature of the substituents bound in the 4-position and optionally in the 3-position of the spirodecane skeleton are substantially different.

According to one aspect of the invention, we provide 4-methylene-2-oxo-8-azasprio[4,5]decane derivatives of formula (I), 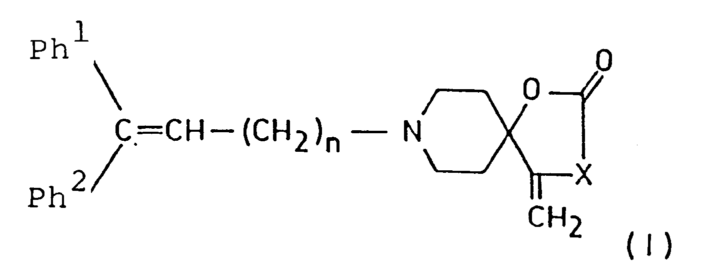
wherein
- X: represents an oxygen atom or a NR group, wherein
R represents a hydrogen atom or a C₁₋₁₂alkyl or C₃₋₆cycloalkyl group,
or a carbocyclic C₆₋₁₀aryl or carbocyclic C₆₋₁₀aryl-C₁₋₄alkyl group optionally substituted on the aromatic ring by one or more halogen atoms,
C₁₋₄alkyl groups or C₁₋₄ alkoxy groups.
- Ph¹ and Ph²,: which may be the same or different, each represent a phenyl group unsubstituted or substituted by one or more halogen atoms or C₁₋₄ alkyl, C₁₋₄alkoxy, trihalomethyl groups or hydroxyl groups optionally esterified by a C₁₋₄alkanoic acid; and
- n: is 1 or 2;

and optical isomers and mixtures thereof, and all solvates, hydrates, acid addition and quaternary ammonium salts thereof.

The compounds of the formula (I) may exist in various stereoisomeric forms such as geometrical isomers as well as racemates, individual (separated) optical isomers and their mixtures, all of which may also occur in the form of various solvates and hydrates. All these compounds and mixtures are within the scope of the present invention.

According to another aspect of the invention, there is provided a process for the preparation of the compounds of the formula (I) as well as their acid addition and quaternary ammonium salts, which comprises
a) for the preparation of compounds of formula (I) wherein X is a group NR, reacting a compound of formula (II), 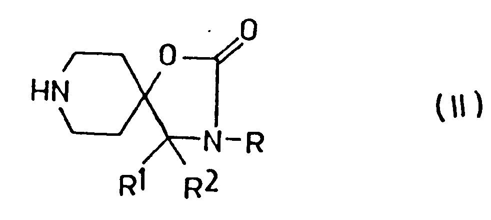 wherein R is as hereinbefore defined and R¹ and R² together are a methylene group, with a compound of formula (III), 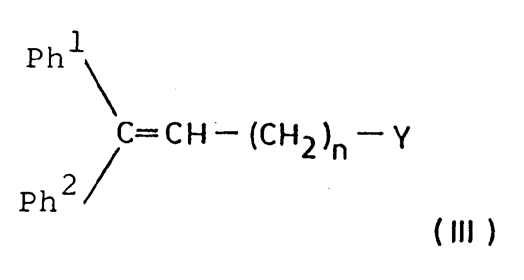 wherein Ph¹ Ph² and n are as hereinbefore defined and Y represents a halogen atom or a C₁₋₄alkylsulfonyloxy or arylsulfonyloxy group;
   or
b) reacting a compound of formula (IV), 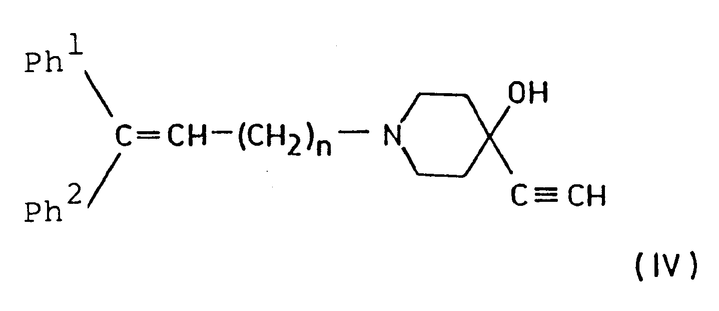 wherein Ph¹, Ph² and n are as defined above, with an isocyanate of formula R-NCO, wherein R is as defined above other than hydrogen,

followed by either
α) for the preparation of compounds of formula (I) wherein X is oxygen, cyclizing the so-obtained compound of formula (V), 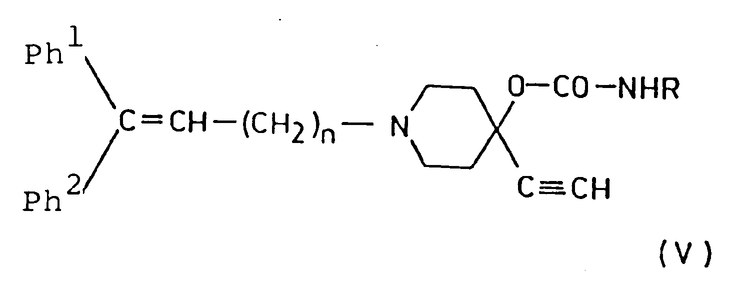
wherein R, Ph¹, Ph² and n are as defined above, in an acidic medium and reacting the resultant compound of formula (VI), 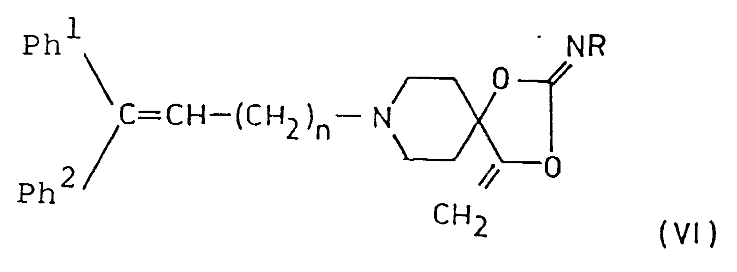
wherein R, Ph¹, Ph² and n are as defined above, obtained in a salt form, with water;
or
β) for the preparation of compounds of formula (I) wherein X is a group NR, cyclizing the thus-obtained compound of formula (V) as defined above, in a basic medium;
or
c) for the preparation of compounds of formula (I) wherein X is oxygen, cyclizing a compound of formula (V), as defined above in an acidic medium and reacting the resultant compound of formula (VI) as defined above, obtained in a salt form, with water;
   or
d) for the preparation of compounds of formula (I) wherein X is a group NR, cyclizing the thus-obtained compound of formula (V) as defined above, in a basic medium;
   or
e) for the preparation of compounds of formula (I) wherein X is a group NR, dehydrating a compound of formula (VII),

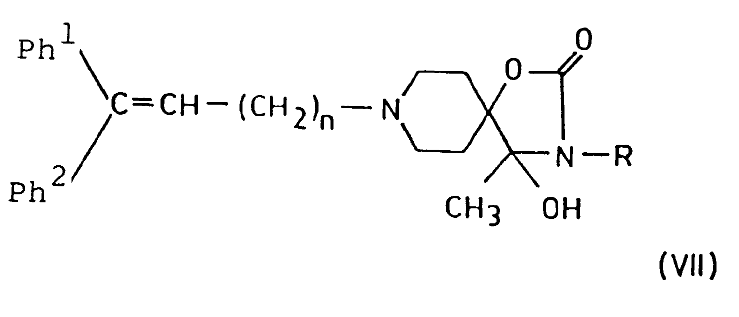

wherein R, Ph¹, Ph² and n are as defined above;
followed if desired or necessary,
by transforming a functional group of a thus-obtained compound of the formula (I), as defined above into another compound of formula (I) as hereinbefore defined in a known manner,
and/or
reacting a thus-obtained compound of the formula (I) as defined above with an acid to give an acid addition salt and/or reacting a salt of compound of the formula (I) as defined above with a base to liberate the free basic form thereof and/or converting a thus-obtained compound of the formula (I) as defined above into its quaternary ammonium salt.

In the process a) according to this invention, a 2-oxo-3,8-diazaspiro[4,5]decane derivative of the formula (II) is reacted with a diphenylalkene derivative of the formula (III), wherein Y may represent a mesyloxy or tosyloxy group or a halogen atom, preferably chlorine or bromine. This reaction is preferably accomplished in an inert organic solvent and preferably in the presence of a base capable of binding the acid liberated in the reaction. Suitable solvents include aliphatic alkanols such as ethanol, isopropanol or butanol; aromatic hydrocarbons such as chlorobenzene or toluene; ethers such as dibutyl ether or dioxan; tertiary aliphatic acid amides such as dimethylformamide, dimethylacetamide; ketones such as acetone, methyl ethyl ketone or methyl isobutyl ketone; or any mixture of the above solvents.
For binding the acid liberated in the reaction, inorganic or tertiary organic bases, e.g. carbonates or hydrogen carbonates of alkaline metals or alkaline earth metals as well as organic bases, e.g. triethylamine, dimethylaniline or pyridine may be used. An excess of the compound of the formula (II) is also suitable for this purpose. This reaction may be carried out between room temperature and the boiling point of the reaction mixture. Optionally, a catalyst may also be added. Suitable catalysts include alkaline metal iodides. It is preferable to work under an inert gas such as nitrogen or argon.

According to the process b) above, a 4-ethynyl-4-hydroxypiperidine derivative of the formula (IV) is first brought into reaction with an isocyanate of the formula R-NCO in a known manner [e.g.Houben-Weyl: Methoden der Organischen Chemie, Vol. VIII/3, page 137 to 147 (1952)] to give a 4-carbamoyloxy-4-ethynylpiperidine derivative of the formula (V). According to step α), the resultant compound of formula (V) is cyclized in an acidic medium and the 2-imino-1,3-dioxolane derivative of formula (VI) obtained as a salt is reacted with water to give compounds of the formula (I) wherein X is oxygen. Or, according to step β), the resultant compound of formula (V) is cyclized in a basic medium to obtain compounds of the formula (I), wherein X stands for a 〉NR group.

The cyclization according to step α) may be carried out in an inert organic solvent (i.e. in a solvent which is inert under the reaction conditions), in the presence of a suitable acid, peferably in the presence of a dry hydrogen halide. Aliphatic or alicyclic ethers such as diethyl ether, dipropyl ether, diisopropyl ether, dibutyl ether, tetrahydrofuran or dioxan as well as lower aliphatic carboxylic acids, e.g. acetic or propionic acid, may be employed as solvents.

As a hydrogen halide hydrogen chloride, bromide, iodide or fluoride, preferably hydrogen chloride or bromide are used. After reaction with water the thus formed 2-imino-1,3-dioxolane hydrohalide salt, the 4-methylene-2-oxo-8-aza-spiro[4,5]decane derivative of the formula (I),is obtained as an acid addition salt, from which, if desired, the base may be liberated in a manner known per se.

The cyclization according to step β) may be carried out in the presence of a base. Alkaline metal acetates, carbonates, alkoxides, hydroxides and/or tertiary organic bases, e.g. pyridine, tripropylamine or picoline, may be used as basic catalysts in the cyclization; the organic bases may also serve as solvents for the reaction. Further suitable solvents include aliphatic alcohols such as methanol, ethanol, propanol or butanol; aliphatic, alicyclic or aromatic hydrocarbons such as methylene chloride, hexane, cyclohexane, benzene, toluene or xylene; acid amides such as dimethylformamide or N-methylpyrrolidone; ethers such as dibutyl ether or dioxane; nitriles such as acetonitrile; sulfoxides, e.g. dimethyl sulfoxide; as well as any mixture of the above solvents. The reaction may also be carried out without any solvent, e.g. in molten state. In order to accelerate the cyclization the temperature may be suitably raised. The reaction is preferably accomplished between 40°C and the boiling point of the reaction mixture. It is suitable to work under an inert gas such as argon or nitrogen. According to a preferred embodiment of the present invention, the 4-carbamoyloxy-4-ethynylpiperidine derivative of the formula (V) obtained from the reaction of 4-ethynyl-4-hydroxypiperidine derivative of the formula (IV) with the isocyanate of the formula R-NCO is not isolated but cyclized directly in the same reaction mixture in the presence of a suitable base, i.e. the derivative of formula (V) is not isolated prior to cyclization.

For processes c) and d) above, the procedures as discussed for steps i) and ii) above may be followed.

In the case of process e) above, a 2-oxo-1-oxa-8-azaspiro[4,5]decane derivative of the formula (VII) is dehydrated. The dehydration may be realized under atmospheric or reduced pressure by using procedures commonly known from the literature. Isocyanates, aliphatic carboxylic acids, aliphatic or aromatic carboxylic acid anhydrides, Lewis acids, sulfuric acid or aromatic sulfonic acids may be employed for dehydration. This reaction is preferably performed in an organic solvent. Suitable solvents include aromatic hydrocarbons such as benzene, toluene or xylene; ethers such as dioxan or di-n-butylether; or aliphatic carboxylic acids such as acetic acid. Optionally, the water formed in the reaction may be distilled off azeotropically.

If desired, the compounds of the formula (I) obtained by using the processes a) to e) may be transformed in a known manner into other compounds of formula (I) within the scope of the present invention.

If desired, the compounds of the formula (I) may be converted into their acid addition and quaternary ammonium salts by using know methods. For the preparation of acid addition salts inorganic or organic acids such as hydrogen halides, e.g. hydrochloric acid or hydrobromic acid; sulfuric acid, phosphoric acids as well as formic, acetic, propionic, oxalic, glycolic, maleic, fumaric, succinic, tartaric, ascorbinic, citric, malic, salicylic, lactic, benzoic, cinnamic, aspartic, glutamic, N-acetyl-aspartic or N-acetylglutamic acid as well as alkanesulfonic acids such as methanesulfonic acid or arenesulfonic acids, e.g. p-toluenesulfonic acid, may be used.

Salt formation may be carried out e.g. in such a way that the corresponding acid is added to the solution of the compound of the formula (I) prepared in an inert solvent, e.g. ethanol, and the salt formed is precipitated by adding preferably a water-immiscible organic solvent, e.g. diethyl ether.

For the preparation of quaternary ammonium salts a lower alkyl, alkenyl or benzyl halide or an alkyl sulfate may preferably be employed. The quaternization may be performed in an organic solvent such as acetone, acetonitrile, ethanol or their mixtures at a temperature in the range from room temperature up to the boiling point of the solvent.

The acid addition or quaternary ammonium salt obtained may be isolated e.g. by filtration and, when necessary, purified by recrystallization.

Conversely, the corresponding free bases can be liberated from their salts by an alkaline treatment.

The compounds of the formulae (IV), (V) and (VII) used as starting substances are also novel and possess their own biological activity.

The compounds of the formula (III) may be prepared according to e.g. Ber. 55, 3406 (1922); Ann. Chem. 555, 80 (1952); GB patent specification No. 683,950; Yakugaku Zasshi 82, 1088 (1952); J. Chem. Soc. 4066 (1959); Coll. Czechoslov. Chem. Commun. 38, 3879 (1973).

The preparation of compounds of formula (II) is described in our EP-A-0412820 of even date claiming priority from Hungarian patent application No. 4092/89.

This preparation comprises the reduction of compounds of formula (X) 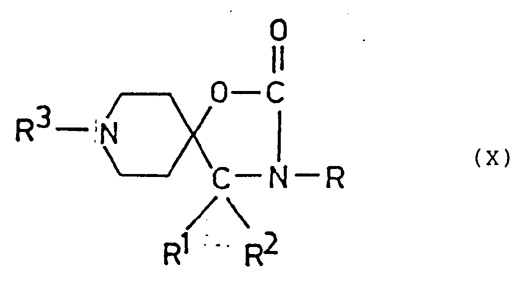
wherein R₁ and R₂ are as defined above and R³ represents a benzyl, (C₁₋₄ alkoxy)carbonyl, phenoxycarbonyl, formyl, piperidin-1-ylcarbonyl, morpholin-4-ylcarbonyl, 4-methylpiperazin-1-ylcarbonyl, 4-(2-hydroxyethyl)piperazin-1-ylcarbonyl, 2-chloro-3-nicotinoylcarbamoyl or C₁₋₆alkylcarbamoyl group.

The compounds of formula (X) may be prepared as also described in the above-mentioned application by reacting a compound of formula (XI) 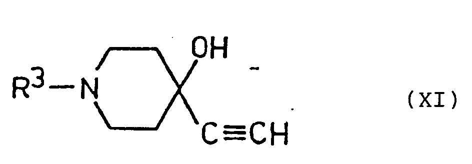
wherein R³ is as defined-above with an isocyanate of formula RNCO wherein R is as hereinbefore defined followed by cyclizing the resulting compound in a basic medium.

Compounds of formula (XI) may be prepared by a procedure analogous to the preparation of compounds of formula (IV) as described below.

The compounds of the formula (IV) can be prepared by the ethynylation reaction of suitably substituted 4-piperidone derivatives as described in e.g. the Hungarian patent specification No. 166,769 or in Farmaco (Pavia) Ed. Sci. 12, 34 (1957).

The carbamates of the formula (V) are obtained by e.g. reacting a compound of the formula (IV) with an isocyanate of the formula R-NCO, e.g. as described above.

The 2-oxo-1-oxa-8-azaspiro[4,5]decane derivatives of the formula (VII) are the subject of our co-pending European Patent Application No. 90308779.9 , also claiming priority of Hungarian Patent Application No. 4094/89, of even date herewith. These can be prepared according to e.g. the process a) described above by reacting a compound of the formula (II) containing a group as defined above for R, a methyl group as R¹ and a hydroxyl group as R², with a suitably substituted compound of formula (III) as defined above.

The compounds of formula (II) wherein R¹ is a methyl group and R² is a hydroxyl group may be prepared as described in our above EP-A-0412820 claiming priority of HU 4092/89, e.g. by hydrating a compound of formula (X) wherein R¹ and R² together form a methylene group.

The compounds of the formula (I) according to the present invention and their salts exert a strong and selective dopaminergic action in the central nervous system, e.g. they inhibit the central dopamine (hereinafter: DA) receptors in the cortical and subcortical brain regions and therefore, they possess antipsychotic effect. Thus, the compounds of the formula (I) are useful for the treatment of various psychiatric disorders such as acute and chronic schizophrenia, maniac-depressive psychosis, agitations of various origin, psychomotor disquiet and other psychoses.

At present several in vivo methods are known for the investigation of inhibition of the cerebral DA receptors. One of these methods is based on the characteristic property of antipsychotic compounds, i.e. that they are capable of inhibiting the e.g. behavioural forms induced in rats by apomorphine which is a DA agonist. It has been proven in a number of studies and investigations that an excellent correlation exists between the in vivo inhibition of DA receptors measured in the apomorphine test and the clinical-therapeutic efficiency of antipsychotic compounds. Apomorphine induces a characteristic syndrome in rats and various animal species which manifests itself in the hyperactivity and stereotypic behaviour of the animals [J. Pharm. Pharmacol. 19 627 (1957); J. Neurol. Transm. 40, 97 (1977); J. Psychiat. Res. 11, 1 (1974); J. Pharm. Pharmacol. 25, 1003 (1973); as well as Nature 263, 338 (1976)].

Male Hannover-Wistar rats weighing 160 to 180 g were used in the present tests. The test compounds were suspended in a 2% Tween 80 solution and diluted to the desired concentration by adding distilled water. The corresponding dose was administered to rats in a volume of 5 ml/kg. The control group was treated with the above solution containing no test substance.

One hour following a treatment with a 2.5 mg/kg oral dose of the test compound, the rats were subcutaneously treated wiht 1 mg/kg of apomorphine hydrochloride.

15 minutes after administration of apomorphine, the animals were placed in a 5-channel behaviour-observing device controlled by a microprocessor and the coordinated and stereotypic motion of the animals were measured for 15 minutes. Chlozapine (chemically 8-chloro-11-(4-methyl-1-piperazinyl)-5H-dibenzo[b,e][1,4]diazepine) was used in a dose of 2.5 mg/kg as a reference drug. The results are shown in the Table below as percentages of the control for both motion types.

Also, the cataleptogenic (catalepsy-inducing) effect of the compounds was investigated by using the method of G. Stille and H. Launer [Arzneim.-Forsch. 21, 252 (1971)]. In these tests male Wistar rats weighing 90 to 110 g were used and were orally treated by various doses of the test substances. The number of cataleptic animals was registered hourly for 6 hours following the treatment. An animal was considered to be cataleptic when it did not correct within 30 seconds its unusual body position caused by lifting its upper limbs onto a horizontal rod set at a height of 8 cm. The ED₅₀ value was calculated from the percentage of cataleptic animals. The results are summarized in following Table.

The abbreviations used in the Table are as follows:
- LMA :: locomotor activity
- n :: number of animals
- p.o.:: oral administration
- S.E.:: standard error of the mean value
- A :: 8-[4,4-bis(4-fluorophenyl)-3-butenyl]3-methyl-4-methylene-2-oxo-1-oxa-3,8-diazaspiro[4,5]decane
- B :: 8-[4,4-bis(4-fluorophenyl)-3-butenyl]-3-butyl-4-methylene-2-oxo-1-oxa-3,8-diazaspiro[4,5]decane

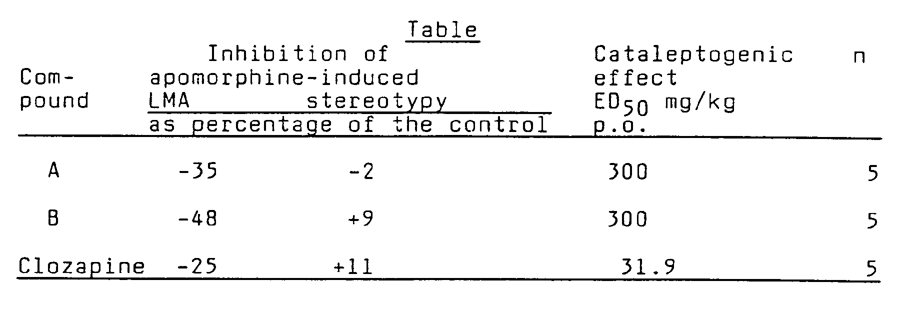

- Control:: LMA: 100% (438.9 ± 54.7 sec ± S.E.)
Steoreotypy: 100% (105.0 ± 13.7 sec ± S.E.)

It is obvious from the Table that a 2.5 mg/kg oral dose of the compounds of the formula (I)according to the present invention decreased the apomorphine-induced locomotor hyperactivity with the same or a significantly higher efficiency than the reference drug, whereas, similarly to clozapine, they did not inhibit the stereotypy. Their cataleptogenic effect was at least ten times as favourable than that of the reference drug. Thus, it can be expected that the extrapyramidal side effects of the compounds of the formula (I) according to the present invention would be less frequent or even absent.

Due to their antipsychotic efficiency, the compounds of the formula (I) may be used in the manufacture of a medicament for the systemic treatment of mammals, including man, suffering from a psychotic disease. Here the term "systemic treatment" means oral, rectal or parenteral administration. Depending on the severity of the disease and the condition of the patient the dose may be varied from 0.01 mg/kg to 40 mg/kg.

The compounds according to the invention may be formulated into pharmaceutical compositions. These compositions may be administered orally, rectaly and/or parenterally. For oral administration, the composition may be formulated e.g. as a tablet, dragée or capsule. In order to prepare oral compositions, e.g. lactose or starch may be used as carriers. Gelatine, sodium carboxymethylcellulose, methylcellulose, polyvinylpyrrolidone or starch gum are suitable binding or granulating agents. As disintegrating agents mainly potato starch or microcrystalline cellulose may be added though ultraamylopectin or formaldehyde-casein are also useful. Talc, colloidal silicic acid, stearin, calcium or magnesium stearate are suitable anti-adhesive and sliding agents. Liquid oral compositions can be formulated as e.g. suspensions, syrups or elixirs which may contain water, glycols, oils, alcohols as well as colouring and flavouring agents.

Tablets may be prepared by e.g. compression following wet granulation. The mixture of the active ingredient with the carriers and optionally with a part of the disintegrating additive is granulated with an aqueous, alcoholic or aqueous-alcoholic solution of the binding agents then the granulate is dried. Subsequently, after mixing the other disintegrating, sliding and anti-adhesive additives to the dried granulate, the mixture is compressed into tablets. If desired, the tablets may be provided with a groove in order to facilitate their administration. Tablets may also be directly prepared from a mixture containing the active ingredient and suitable additives. The tablets may optionally be converted to dragées by employing commonly used pharmaceutical additives, e.g. protective, flavouring or colouring agents such as sugar, cellulose derivatives (methyl- or ethylcellulose, sodium carboxymethylcellulose), polyvinylpyrrolidone, calcium phosphate, calcium carbonate, food dyes, dyeing lacquers, aromatizing agents, iron oxide, and pigments. Encapsulated compositions are prepared by filling a mixture of the active ingredient with the additives into capsules.

For rectal administration, the composition of the invention may be formulated as a suppository containing a carrier mass, the so-called "adeps pro suppositorio", in addition to the active ingredient. As carriers, vegetable fats such as hardened vegetable oils, or triglycerides of C₁₂₋₁₈ fatty acids (preferably the carriers bearing the trade name Witepsol) may be used. The active ingredient is uniformly distributed in the molten carrier mass, then suppositories are prepared by moulding.

For parenteral administration, the composition of the invention may be formulated as an injectable solution. For preparing these injectable solutions, the active ingredients are dissolved in distilled water and/or various organic solvents, e.g. glycol ethers, if desired, in the presence of solubilizing agents such as polyoxyethylene sorbitan monolaurate or monooleate or monostearate (Tween 20, Tween 60 or Tween 80), respectively. The injectable solution may further contain various auxiliary agents, e.g. preservatives such as ethylenediamine tetraacetate as well as pH-modifying and buffering substances or, if desired, a local anaesthetic agent such as lidocaine. Before filling into the ampoules, the injectable solution containing the composition of the invention is filtered and after filling in, it is subjected to sterilization.

The invention is illustrated by way of the following Preparations and Examples.

### Preparation 1

### 4-methylene-2-oxa-3-n-propyl-1-oxa-3,8 diazaspiro [4,5] decane

### (a) 8-benzyl-3-n-butyl-4-methylene-2-oxo-1-oxa-3,8-diazaspiro[4,5]decane

21.5 g of 1-benzyl-4-ethynyl-4-hydroxypiperidine are boiled under reflux with 12.9 g of n-butyl isocyanate in the presence of 0.4 g of anhydrous potassium acetate in 66 ml of 2-picoline under nitrogen for 6 hours. After evaporating 2-picoline under reduced pressure and dissolving the residue in benzene, the organic solution is washed with water and dried over anhydrous magnesium sulfate. After filtration on an aluminum oxide layer the benzene solution is evaporated under reduced pressure. The crude product obtained is recrystallized from n-heptane to give the pure title substance in 78.5% yield, m.p.: 57-58 °C.

### Analysis:

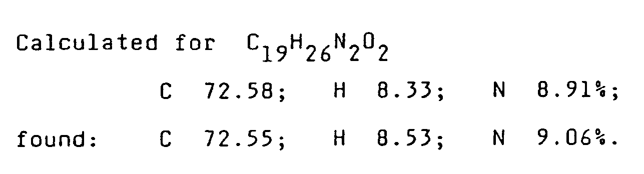

Using the appropriate starting materials the following compounds may be prepared in an analogous manner;
8-Formyl-4-methylene-2-oxo-3-phenyl-1-oxa-3,8-diazaspiro[4,5]decane, m.p.: 171-172 °C;
8-Benzyloxycarbonyl-4-methylene-2-oxo-3-phenyl-1-oxa-3,8-diazaspiro[4,5]decane, m.p.: 145-146 °C;
4-Methylene-2-oxo-3-phenoxycarbonyl-3-phenyl-1-oxa-3,8-diazaspiro[4,5]decane, m.p.: 208-210 °C;
8-Benzyl-3-cyclohexyl-4-methylene-2-oxo-1-oxa-3,8-diazaspiro[4,5]decane, m.p.: 128-130 °C (the hydrogen fumarate salt melts at 207-208 °C);
8-Benzyl-3-ethyl-4-methylene-2-oxo-1-oxa-3,8-diazaspiro[4,5]decane, m.p.: 103-104 °C (hydrogen maleate salt m.p.: 184-186 °C);
8-Benzyl-3-tert-butyl-4-methylene-2-oxo-1-oxa-3,8-diazaspiro[4,5]decane, m.p.: 116-117 (dihydrogen citrate salt m.p.: 132-133 °C);
8-Benzyl-4-methylene-2-oxo-3-phenyl-1-oxa-3,8-diazaspiro[4,5]decane, m.p.: 134-135 °C;
8-Benzyl-3-isopropyl-4-methylene-2-oxo-1-oxa-3,8-diazaspiro[4,5]decane, m.p.: 96-97 °C;
8-Benzyl-3-methyl-4-methylene-2-oxo-1-oxa-3,8-diazaspiro[4,5]decane hydrogen maleate, m.p.: 210-211 °C;
8-Benzyl-4-methylene-2-oxo-3-propyl-1-oxa-3,8-diazaspiro[4,5]decane dihydrogen citrate, m.p.: 168-171 °C;

### (b) 3-tert-butyl-4-methylene-2-oxo-8-phenoxycarbonyl-1-oxa-3,8-diazaspiro[4,5]decane

A solution of 3.6 g of phenyl chloroformate in 5 ml of methylene chloride are dropped into a solution of 6.3 g of 8-benzyl-3-tert-butyl-4-methylene-2-oxo-1-oxa-3,8-diazaspiro[4,5]decane in 30 ml of methylene chloride under argon at 0 °C while stirring, then the reaction mixture is stirred at room temperature for one additional hour. After termination of the reaction the mixture is diluted with 35 ml of methylene chloride, extracted with 4 N sodium hydroxide solution and washed to neutral with water. After drying over anhydrous magnesium sulfate the solvent is evaporated under reduced pressure. After adding n-hexane to the residue the solid precipitate is filtered off and recrystallized from isopropanol to obtain the title substance in 82% yield, m.p.: 125-126 °C.

### Analysis:

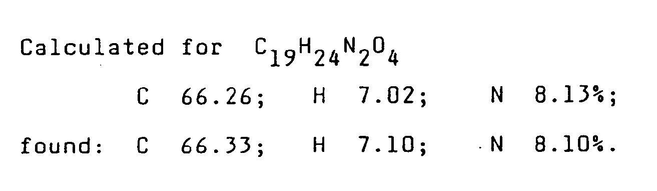

Using the appropriate starting materials the following compounds may be prepared in an analogous manner.
3-Benzyl-8-benzyloxycarbonyl-4-methylene-2-oxo-1-oxa-3,8-diazaspiro[4,5]decane, oil;
8-benzyloxycarbonyl-3-butyl-4-methylene-2-oxo-1-oxa-3,8-diazaspiro[4,5]decane, m.p.: 47-48 °C;
8-ethoxycarbonyl-3-methyl-4-methylene-2-oxo-1-oxa-3,8-diazaspiro[4,5]decane, m.p.: 121-122 °C;
3-(3,4-dichlorophenyl)-4-methylene-2-oxo-8-phenoxycarbonyl-1-oxa-3,8-diazaspiro[4,5]decane, m.p.: 220-222 °C;
3-methyl-4-methylene-2-oxo-8-phenoxycarbonyl-1-oxa-3,8-diazaspiro[4,5]decane, m.p.: 118-119 °C; and
4-methylene-2-oxo-8-phenoxycarbonyl-3-propyl-1-oxa-3,8-diazaspiro[4,5]decane, m.p.: 96-98 °C;

### (c) 4-methylene-2-oxo-3-n-propyl-1-oxa-3,8-diazaspiro[4,5]decane

A suspension containing 0.5 g of 10% by weight palladium-on-carcoal catalyst in 5 ml of water is added to the solution of 5.0 g of 8-benzyloxycarbonyl-4-methylene-2-oxo-3-n-propyl-1-oxa-3,8-diazaspiro[4,5]decane in 45 ml of methanol at 0 °C under argon while stirring. To this mixture 1 ml of 45.8% aqueous hydrazine solution is introduced and the reaction mixture is refluxed for 10 to 15 minutes. After cooling down to room temperature and filtering off the catalyst the solvent is evaporated under reduced pressure and the crude evaporation residue is recrystallized from benzene to give the title compound in 95% yield, m.p.: 96-97 °C.

Using the appropriate starting materials the following compounds may be prepared in an analogous manner;
3-Ethyl-4-methylene-2-oxo-1-oxa-3,8-diazaspiro[4,5]decane, m.p.: 106-108 °C;
3-isopropyl-4-methylene-2-oxo-1-oxa-3,8-diazaspiro[4,5]-decane, m.p.: 151-152 °C;
4-methylene-3-(1-naphthyl)-2-oxo-1-oxa-3,8-diazaspiro[4,5]-decane, m.p.: 208-209 °C;
3-butyl-4-methylene-2-oxo-1-oxa-3,8-diazaspiro[4,5]decane, oil;
4-methylene-2-oxo-3-phenyl-1-oxa-3,8-diazaspiro[4,5]decane, m.p.: 185-186 °C;
3-tert-Butyl-4-methylene-2-oxo-1-oxa-3,8-diazaspiro[4,5]decane, m.p.: 138-139 °C;
3-heptyl-4-hydroxy-4-methyl-2-oxo-1-oxa-3,8-diazaspiro[4,5]decane, m.p.: 139-140 °C;
3-[2-(3,4-dimethoxyphenyl)ethyl-]-4-hydroxy-4-methyl-2-oxo-1-oxa-3,8-diazaspiro[4,5]decane, m.p.: 190-191 °C;
3-benzyl-4-methylene-2-oxo-1-oxa-3,8-diazaspiro[4,5]decane, m.p.: 77-79 °C;
3-heptyl-4-methylene-2-oxo-1-oxa-3,8-diazaspiro[4,5]decane, oil;
3-decyl-4-methylene-2-oxo-1-oxa-3,8-diazaspiro[4,5]decane, oil;
3-cyclohexyl-4-methylene-2-oxo-1-oxa-3,8-diazaspiro[4,5]decane, m.p.: 141-142 °C;
3-[2-(3,4-dimethoxyphenyl)ethyl]-4-methylene-2-oxo-1-oxa-3,8-diazaspiro[4,5]decane, m.p.: 107-109 °C.

### Preparation 2

### 3,4-dimethyl-4-hydroxy-2-oxo-1-oxa-3,8-diazaspiro[4,5]decane hydrochloride

A solution of 5.44 g of 8-benzyl-3-methyl-4-methylene-2-oxo-1-oxa-3,8-diazaspiro[4,5]decane (from Preparation 1(a)) in 40 ml of N hydrochloric acid is hydrogenated in the presence of 5.4 g of palladium-on-charcoal catalyst until the theoretical amount of hydrogen is consumed. After filtering off the catalyst the filtrate is evaporated to a volume of about 10 ml under reduced pressure and 40 ml of acetone are added. The crystalline precipitate is filtered off and dried to obtain the title compound in 87% yield, m.p.: 272-274 °C.

### Analysis of the base:

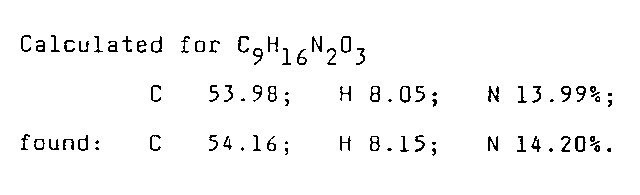

### Preparation 3

### 8-[3,3-bis(4-fluorophenyl)-2-propenyl]-4-hydroxy-4-methyl-2-oxo-3-n-propyl-1-oxa-3,8-diazaspiro[4,5]-decane

9.3 g of 3,3-bis(4-fluorophenyl)-2-propenyl bromide dissolved in 50 ml of acetone are added portionwise to a mixture containing 6.85 g of 4-hydroxy-4-methyl-2-oxo-3-n-propyl-1-oxa-3,8-diazaspiro[4,5]decane (prepared analogously to Preparation 2 above) and 4.2 g of anhydrous potassium carbonate in 68 ml of anhydrous acetone at room temperature for 1 hour while stirring, then the reaction mixture is stirred at room temperature for an additional hour. After filtering off the inorganic salts and evaporating the solvent under reduced pressure, the residue is taken up in benzene, washed with water, dried over anhydrous sodium sulfate and then the solution is evaporated to a tenth of its volume under reduced pressure. The product is precipitated by adding n-hexane to the evaporation residue. The crystals are filtered off and dried to give the title compound in 86% yield, m.p.: 128-129°C.

### Analysis:

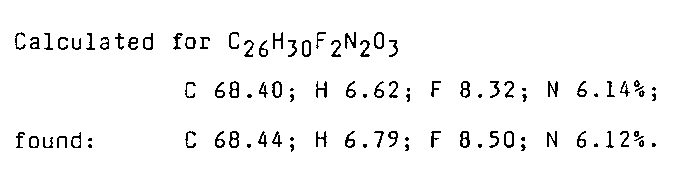

The hydrochloride salt is precipitated by adding ethereal hydrogen chloride solution to an ethereal solution of the base, m.p.: 233-235°C.

The following compounds may be prepared analogously using the process as described in the preceding Preparation and using the appropriate starting materials:
8-(3,3-diphenyl-2-propenyl)-4-hydroxy-3-isopropyl-4-methyl-2-oxo-1-oxa-3,8-diazaspiro[4,5]decane hydrochloride, m.p.: 219-221°C;
8-[3,3-bis(4-fluorophenyl)-2-propenyl]-4-hydroxy-3-isopropyl-4-methyl-2-oxo-1-oxa-3,8-diazaspiro[4,5]decane hydrochloride, m.p.: 255-260°C.

### Example 1

### 8-[4,4-bis(4-fluorophenyl)-3-butenyl]-4-methylene-2-oxo-3-phenyl-1-oxa-3,8-diazaspiro[4,5]decane

A mixture containing 8.4 g of 4-methylene-2-oxo-3-phenyl-1-oxa-3,8-diazaspiro[4,5]decane, 18.4 g of 4,4-bis(4-fluorophenyl)-3-butenyl chloride, 0.3 g of potassium iodide, 9,2 g of anhydrous potassium carbonate and 81 ml of methyl isobutyl ketone is mildly refluxed under argon while stirring for 12 hours. After cooling down and filtering off the inorganic salts, the precipitate is washed with methyl isobutyl ketone, the filtrate is washed with water to neutral, dried over anhydrous magnesium sulfate, then the solvent is evaporated under reduced pressure. After triturating the evaporation residue with n-hexane, the precipitate is filtered and recrystallized from ethanol to give the title compound in 83.5% yield, m.p.: 136.5-137.5°C.

### Analysis:

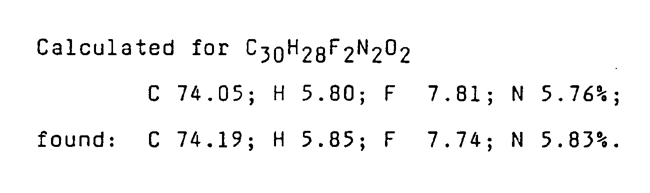

### Example 2

### 8-[4,4-bis(4-fluorophenyl)-3-butenyl]-3-ethyl-4-methylene-2-oxo-1-oxa-3,8-diazaspiro[4,5]decane

A mixture containing 7.8 g of 3-ethyl-4-methylene-2-oxo-1-oxa-3,8-diazaspiro[4,5]decane, 8.5 ml of anhydrous triethylamine, 80 ml of anhydrous methyl ethyl ketone, 0.3 g of potassium iodide and 16.7 g of 4,4-bis(4-fluorophenyl)-3-butenyl chloride is refluxed under nitrogen while stirring for 15 hours, then the solvent is distilled off under reduced pressure. After adding benzene and water to the residue, the benzene layer is washed with water to neutral, dried over anhydrous magnesium sulfate, filtered through an aluminum oxide bed and evaporated under reduced pressure. After recrystallizing the residue from ethanol, the title product is obtained in 68.7% yield, m.p.: 138-139°C.

### Analysis:

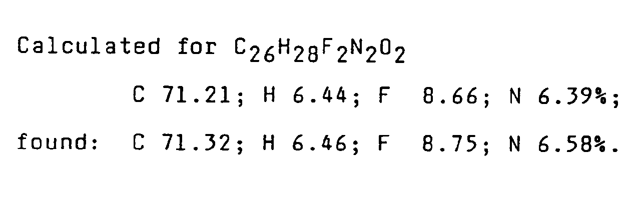

### Example 3

### 8-(3,3-diphenyl-2-propenyl)-3-methyl-4-methylene-2-oxo-1-oxa-3,8-diazaspiro[4,5]decane

9,4 g of 1-(3,3-diphenyl-2-propenyl)-4-ethynyl-4-methylcarbamoyloxypiperidine are refluxed in 100 ml of 0.05 mol/litre ethanolic sodium ethoxide solution under argon for 3 hours. After cooling down, the alkoxide is decomposed by adding aqueous ammonium chloride solution and the solvent is distilled off under reduced pressure. After adding benzene and water to the residue, the benzene layer is separated and dried, then evaporated under reduced pressure. After recrystallizing the solid evaporation residue from ethanol, the title compound is obtained in 71.7% yield, m.p.: 126-127°C.

### Analysis:

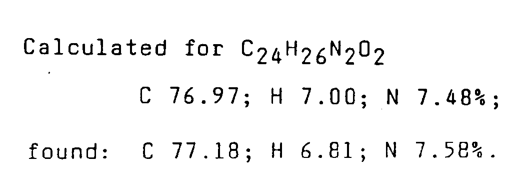

The following compound may be prepared in a manner analogous to the process described in Example 3 above using the appropriate starting materials: 8-(3,3-diphenyl-2-propenyl)-3-isopropyl-4-methylene-2-oxo-1-oxa-3,8-diazaspiro[4,5]decane, m.p. 131-132°C.

### Example 4

### 8-[4,4-bis(4-fluorophenyl)-3-butenyl]-3-cyclohexyl-4-methylene-2-oxo-1-oxa-3,8-diazaspiro[4,5]decane

20.4 g of 8-[4,4-bis(4-fluorophenyl)-3-butenyl]3-cyclo-hexyl-4-hydroxy-4-methyl-2-oxo-1-oxa-3,8-diazaspiro[4,5]decane (prepared in a manner analogous to the process described in Preparation 3 above using the appropriate starting material) are boiled with 1.52 g of p-toluenesulfonic acid monohydrate in 204 ml of xylene while stirring in an equipment fitted with a water-trap and azeotropically distilling off the water formed in the reaction. After the termination of the reaction (about 2 hours) the reaction mixture is cooled down and its pH value is adjusted to 9-10 by adding 5% by weight aqueous sodium hydroxyde solution. Then, the organic phase is washed with water to neutral, dried over anhydrous sodium sulfate and evaporated under reduced pressure. After recrystallizing the residue from a mixture of benzene and ethanol, the title product is obtained in 90.3% yield, m.p.: 145-146°C.

### Analysis:

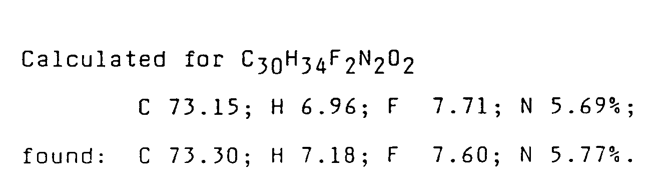

The following compounds may be prepared in a manner analogous to the process described in the preceding Example using the appropriate starting materials.
8-[4,4-bis(4-fluorophenyl)-3-butenyl]-3-decyl-4-methylene-2-oxo-1-oxa-3,8-diazaspiro[4,5]decane, m.p.: 50-51°C;
8-[3,3-bis(4-fluorophenyl)-2-propenyl]-3-isopropyl-4-methylene-2-oxo-1-oxa-3,8-diazaspiro[4,5]decane, m.p.: 137-138°C; and
8-[4,4-bis(4-fluorophenyl)-3-butenyl]-3-[2-(3,4-dimethoxyphenyl)ethyl]-4-methylene-2-oxo-oxa-3,8-diazaspiro[4,5]decane, m.p.: 88-90°C.

### Example 5

### 8-[4,4-bis(4-fluorophenyl)-3-butenyl]-4-methylene-3-(1-naphthyl)-2-oxo-1-oxa-3,8-diazaspiro[4,5]decane

A mixture containing 11.0 g of 1-[4,4-bis(4-fluorophenyl)-3-butenyl]-4-ethynyl-4-hydroxypiperidine, 6.0 g of 1-naphthyl isocyanate, 0.2 g of anhydrous potassium acetate and 36 ml of α-picoline is refluxed under nitrogen while stirring for 5 hours. After evaporating the solvent under reduced pressure, the residue is taken up in benzene, filtered through an aluminum oxide column and then evaporated under reduced pressure. The residue is recrystallized from methanol under clarifying by activated carbon to give the title product in 76.5% yield, m.p.: 99-101°C.

### Analysis:

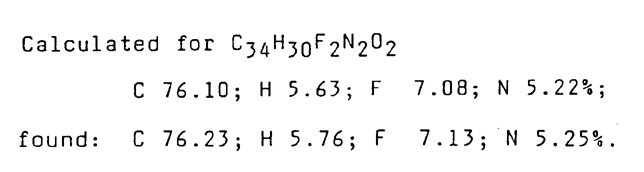

By using the appropriate starting materials, the following compound may be prepared analogously using the process described in the preceding Example:
8-[4,4-bis(4-fluorophenyl)-3-butenyl]-3-isopropyl-4-methylene-2-oxo-1-oxa-3,8-diazaspiro[4,5]decane, m.p. : 135-136°C.

### Example 6

### 8-[4,4-bis(4-fluorophenyl)-3-butenyl]-3-(4-chlorophenyl)-4-methylene-2-oxo-1-oxa-3,8-diazaspiro[4,5]-decane

A solution containing 5.8 g of 4-acetyl-1-[4,4-bis(4-fluorophenyl)-3-butenyl]-4-hydroxypiperidine and 7.0 g of 4-chlorophenyl isocyanate in 30 ml of tripropylamine is gently refluxed under argon while stirring for 3 hours, then the solvent is distilled off under reduced pressure. After adding benzene to the residue and filtering off the insoluble part, the benzene filtrate is filtered through a silica gel bed and evaporated under reduced pressure. The solid evaporation residue is recrystallized from ethanol under clarifying by activated carbon to obtain the title compound in 52% yield, m.p.: 186-188 °C.

### Analysis:

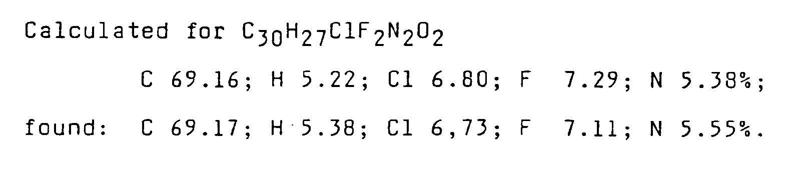

### Example 7

### 8-[4,4-bis(4-fluorophenyl)-3-butenyl]-3-n-butyl-4-methylene-2-oxo-1-oxa-3,8-diazaspiro[4,5]decane

A mixture containing 8.2 g of 8-[4,4-bis(4-fluorophenyl)-3-butenyl]-4-methylene-2-oxo-1,3-dioxa-8-azaspiro[4,5]decane [Example 11 below], 25 ml of xylene and 2.0 ml of butylamine is stirred at room temperature for 18 hours, then 0.8 g of p-toluenesulfonic acid monohydrate and 70 ml of xylene are added and the reaction mixture is boiled while azeotropically distilling off the water formed in the reaction. After termination of the reaction which is observed by thin layer chromatography (TLC), the mixture is cooled down and made alkaline to a pH of 9 to 10 by adding 5% by weight aqueous sodium hydroxide solution. After washing the organic phase with water to neutral and drying over anhydrous magnesium sulfate, the solvent is distilled off under reduced pressure. The crude product obtained as evaporation residue is recrystallized from ethanol under clarifying by activated carbon to give the title product in 72.5% yield, m.p.: 92-93°C.

### Analysis:

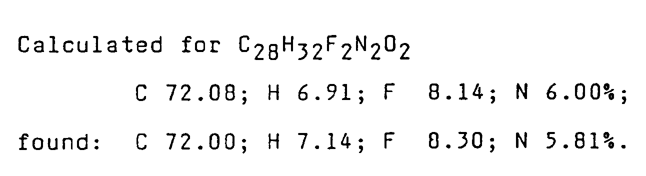

By using the appropriate starting materials the following compound may be prepared analogously using the process described in the preceding Example:
8-[4,4-bis(4-fluorophenyl)-2-propenyl]-4-methylene-2-oxo-3-propyl-1-oxa-3,8-diazaspiro[4,5]decane, m.p.: 128-129°C.

### Example 8

### 8-[4,4-bis(4-fluorophenyl)-3-butenyl]-8-methyl-4-methylene-2-oxo-3-propyl-1-oxa-3,8-diazaspiro[4,5]decane-8-ium iodide

4.0 g of 8-[4,4-bis(4-fluorophenyl)-3-butenyl]-4-methylene-2-oxo-3-propyl-1-oxa-3,8-diazaspiro[4,5]decane are boiled under reflux with 0.7 ml of methyl iodide in 40 ml of methyl isobutyl ketone for 2 hours. After cooling down, the crystalline precipitate is filtered, washed with diisopropyl ether, cooled to 0°C and dried to obtain 92.2% yield of the title quaternary ammonium salt, m.p.: 244-245°C.

### Example 9

### 8-(3,3-diphenyl-2-propenyl)-4-methylene-2-oxo-3-propyl-1-oxa-3,8-diazaspiro[4,5]decane

a) A solution containing 12.6 g of 4-acetyl-1-(3,3-diphenyl-2-propenyl)-4-propylcarbamoyloxypiperidine and 0.4 g of sodium methoxide in 80 ml of methanol is refluxed under argon for 4 to 5 hours, then the solvent is evaporated under reduced pressure. The residue is stirred with 50 ml of 1.0 mol/litre hydrochloric acid at 0 to 5°C for 15 minutes. After filtering off, the crystalline precipitate is washed with ice-cold water and dried to obtain the product hydrochloride in 82.4% yield, m.p.: 132-134°C.
Analysis of the base: 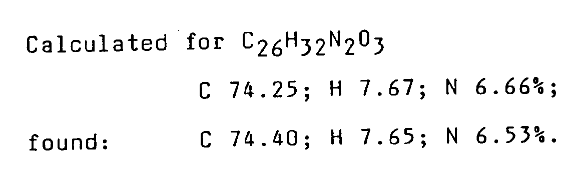
b) 12.6 g of 8-(3,3-diphenyl-2-propenyl)-4-hydroxy-4-methyl-2-oxo-3-propyl-1-oxa-3,8-diazaspiro[4,5]decane are refluxed in a mixture of 126 ml of acetic acid and 5.7 ml of acetic anhydride under nitrogen while stirring for 4 to 5 hours, then the solvent is distilled off under reduced pressure. The residue is made alkaline by adding 5% by weight aqueous sodium hydroxide solution and extracted with benzene. After washing the benzene phase with water to neutral and drying over anhydrous sodium sulfate, benzene is evaporated under reduced pressure and the remaining crude product is recrystallized from ethanol to give the title compound in 85.7% yield, m.p.: 126-127°C.

### Analysis:

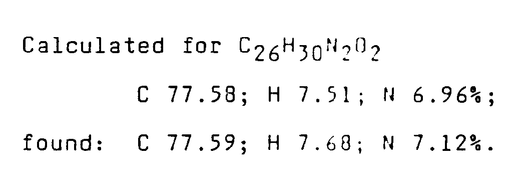

### Example 10

### 8-[4,4-bis(4-chlorophenyl)-3-butenyl]-3-butyl-4-methylene-2-oxo-1-oxa-3,8-diazaspiro[4,5]decane

10.7 g of 4,4-bis(4-chlorophenyl)-3-butenyl bromide are added to the solution of 4.5 g of 3-butyl-4-methylene-2-oxo-1-oxa-3,8-diazaspiro[4,5]decane in 45 ml of acetone containing 4.20 g of anhydrous potassium carbonate and 0.5 g of potassium iodide. The heterogeneous reaction mixture is refluxed under nitrogen while stirring for 5 hours. After evaporating the solvent under reduced pressure, water is added to the residue and extracted with benzene. The benzene phase is washed with water until halide-free, filtered through a silica gel bed and evaporated under reduced pressure. The residue is recrystallized from ethanol to give the title product in 79.0% yield, m.p.: 110-111°C.

### Analysis:

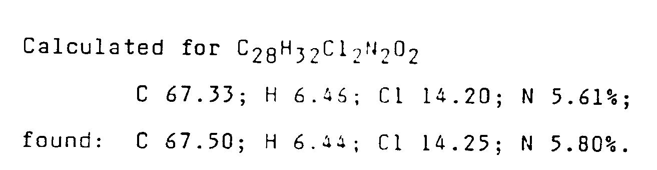

Using appropriate starting materials, the following compounds may be prepared analogously using the process described in the preceding Example :
8-[4,4-bis(4-fluorophenyl)-3-butenyl]-3-methyl-4-methylene-2-oxo-1-oxa-3,8-diazaspiro[4,5]decane, m.p.: 127.5-128.5 °C;
8-[3-(4-acetyloxyphenyl)-3-(3-trifluoromethylphenyl)-2-propenyl]4-methylene-2-oxo-3-phenyl-1-oxa-3,8-diazaspiro[4,5]decane (E:Z=1:1), m.p.: 124-126°C;
8-[3,3-bis(4-fluorophenyl)-2-propenyl]-3-butyl-4-methylene-2-oxo-1-oxa-3,8-diazaspiro[4,5]decane, m.p.: 132.5-134°C;
(E)-8-[3-(acetyloxyphenyl)-3-(3-trifluoromethylphenyl)-2-propenyl]-3-cyclohexyl-4-methylene-2-oxo-1-oxa-3,8-diazaspiro[4,5]decane, m.p.: 177-178°C;
8-[3,3-bis(3,5-dichlorophenyl)-2-propenyl]-3-methyl-4-methylene-2-oxo-1-oxa-3,8-diazaspiro[4,5]decane, m.p.: 141-143 °C;
(E)-8-[3-(4-acetyloxyphenyl)-3-(3-trifluoromethylphenyl)-2-propenyl]-4-methylene-2-oxo-3-propyl-1-oxa-3,8-diazaspiro[4,5]decane, m.p.: 111-112°C;
3-butyl-8-(3,3-diphenyl-2-propenyl)-4-methylene-2-oxo-1-oxa-3,8-diazaspiro[4,5]decane, m.p.: 138-139°C;
3-benzyl-8-[4,4-bis(4-fluorophenyl)-3-butenyl]-4-methylene-2-oxo-1-oxa-3,8-diazaspiro[4,5]decane, m.p.: 78-79°C;
8-[4,4-bis(4-fluorophenyl)-3-butenyl]-3-tert-butyl-4-methylene-2-oxo-1-oxa-3,8-diazaspiro[4,5]decane, m.p.: 109-110 °C;
8-[4,4-bis(4-fluorophenyl)-2-propenyl]-3-methyl-4-methylene-2-oxo-1-oxa-3,8-diazaspiro[4,5]decane, m.p.: 141-142°C;
8-[4,4-bis(4-fluorophenyl)-3-butenyl]-4-methylene-2-oxo-3-propyl-1-oxa-3,8-diazaspiro[4,5]decane, m.p. : 130-131°C;
8-[4,4-bis(4-chlorophenyl)-3-butenyl]-3-methyl-4-methylene-2-oxo-1-oxa-3,8-diazaspiro[4,5]decane, m.p.: 136-137°C; and
3-benzyl-8-[4,4-bis(4-chlorophenyl)-3-butenyl]-4-methylene-2-oxo-1-oxa-3,8-diazaspiro[4,5]decane, m.p.: 138-139°C;

### Example 11

### 8-[4,4-bis(4-fluorophenyl)-3-butenyl]-4-methylene-2-oxo-1,3-dioxa-azaspiro[4,5]decane

After introducing dry gaseous hydrogen chloride into a solution of 32.0 g of 1-[4,4-bis(4-fluorophenyl)-3-butenyl]-4-butylcarbamoyloxy-4-ethynylpiperidine in 160 ml of anhydrous dioxan at 15 to 20°C for 2.5 to 3 hours, the reaction mixture is left to stand overnight. After evaporating the solvent under reduced pressure at 40 to 50°C, water is added to the residue and the crystalline product is filtered off. This hydrochloride precipitate is suspended in water, the base is liberated by adding sodium hydrogen carbonate and extracted into benzene. After drying over anhydrous sodium sulfate, the benzene solution is evaporated under reduced pressure on a water bath of 40°C. The evaporation residue is recrystallized from a mixture of diisopropyl ether and hexane under clarifying by activated carbon to give the title product in 84.4% yield, m.p.: 70.5-72°C.

### Analysis:

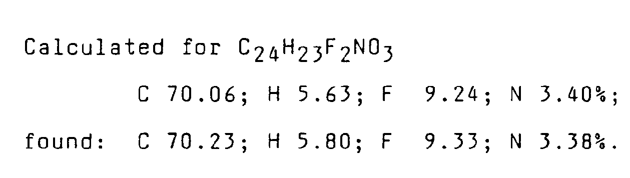

The new compounds according to the present invention may be formulated into the following examples of pharmaceutical compositions:
a) Preparation of tablets
   50.0 g of active ingredient are mixed together with 92 g of lactose, 40 g of potato starch, 4 g of polyvinylpyrrolidine, 6 g of talc, 1 g of magnesium stearate, 1 g of colloidal silicon dioxide (Aerosil) and 6 g of ultraamylopectin and, after wet granulation, the product obtained is compressed into tablets containing 50 mg of the active ingredient each;
b) Preparation of dragées
   After coating the tablets prepared as described above in a known manner with a layer comprising sugar and talc, the dragées obtained are polished with a mixture of bees wax and carnauba wax to obtain dragées weighing 250 mg each;
c) Preparation of capsules
   100 g of active ingredient are thoroughly mixed together with 30 g of sodium lauryl sulfate, 280 g of starch, 280 g of lactose, 4 g of colloidal silicon dioxide (Aerosil) and 6 g of magnesium stearate, then the mixture is sieved and filled into hard gelatine capsules to obtain capsules containing 100 mg of active ingredient each;
d) Preparation of suppositories
   (Note: all weights are calculated for one suppository)
   100.0 mg of active ingredient are thoroughly mixed together with 200.0 mg of lactose, 1700.0 mg of suppository base (e.g. Witepsol 4) are made molten, cooled to 35°C and the mixture of the active ingredient and lactose is mixed thereto by using a homogenizer. The product obtained is poured into cooled conic moulds. Each suppository weighes 2000 mg.
e) Preparation of a suspension
   Components in 100 ml of the suspension:

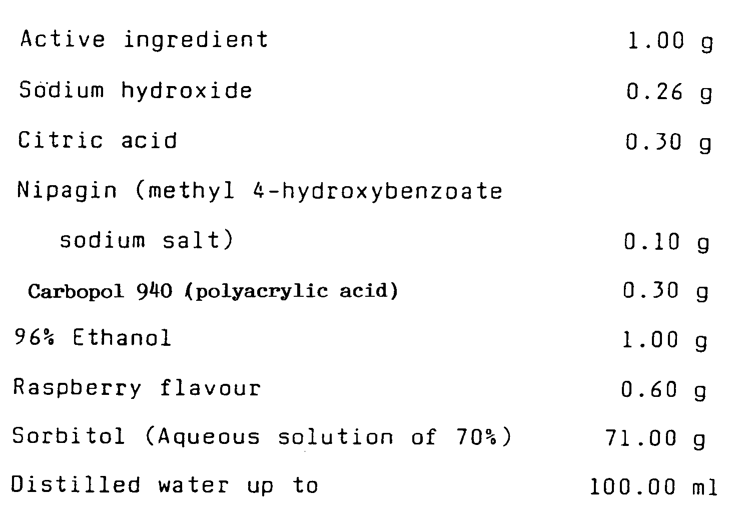

After adding Carbopol in little portions to the solution of Nipagin and citric acid in 20 ml of distilled water under vigorous stirring, the solution obtained is let to stand for 10 to 12 hours. Subsequently, the amount given above of sodium hydroxyde dissolved in 1 ml of distilled water, the aqueous solution of sorbitol and finally the ethanolic solution of the rapsberry flavour are dropped in under stirring. The active ingredient is added in small portions to this mixture and suspended by using a submerged homogenizer. Finally, the suspension is supplemented to 100 ml by adding sufficient distilled water and the syrupy suspension is led through a colloid mill.

"Aerosil", "Witepsol", "Nipagin" and "Carbopol" are all trade marks.

## Claims (Claims for the following Contracting State(s): AT, BE, CH, DE, DK, FR, GB, IT, LI, LU, NL, SE)

1. A compound of formula (I); 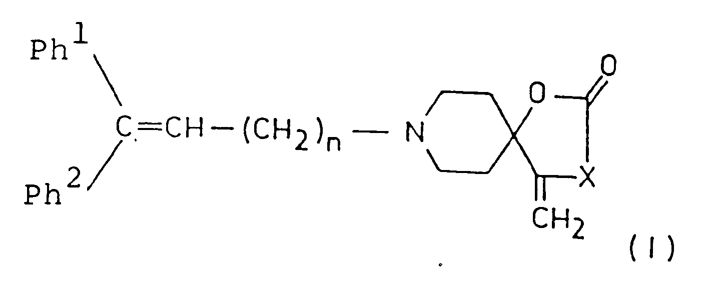 wherein
X represents an oxygen atom or a NR group, wherein
R represents a hydrogen atom or a C₁₋₁₂alkyl or C₃₋₆cycloalkyl group,
or a carbocyclic C₆₋₁₀aryl or carbocyclic C₆₋₁₀aryl-C₁₋₄alkyl group optionally substituted on the aromatic ring by one or more halogen atoms,
C₁₋₄alkyl groups or C₁₋₄alkoxy groups.
Ph¹ and Ph², which may be the same or different, each represent a phenyl group unsubstituted or substituted by one or more halogen atoms or C₁₋₄alkyl, C₁₋₄alkoxy, trihalomethyl groups or hydroxyl groups optionally esterified by a C₁₋₄alkanoic acid; and
n is 1 or 2;
and optical isomers and mixtures thereof, and all solvates, hydrates, acid addition and quaternary ammonium salts thereof.

2. A compound as defined in claim 1 selected from the group comprising:
8-[4,4-bis(4-fluorophenyl)-3-butenyl]-4-methylene-2-oxo-1-oxa-3,8-diazaspiro[4,5]decane,
3-benzyl-8-[4,4-bis(4-fluorophenyl)-3-butenyl]-4-methylene-2-oxo-1-oxa-3,8-diazaspiro[4,5]decane,
8-[4,4-bis(4-fluorophenyl)-3-butenyl]-4-methylene-2-oxo-1,3-dioxa-8-azaspiro[4,5]decane and
8-[4,4-bis(4-fluorophenyl)-3-butenyl]-3-methyl-4-methylene-2-oxo-1-oxa-3,8-diazaspiro[4,5]decane
and optical isomers and mixtures thereof, and all solvates, hydrates, acid addition and quaternary ammonium salts thereof.

3. A pharmaceutical composition which comprises as active ingredient a compound of formula (I) as defined in claim 1 or a pharmaceutically acceptabLe acid addition or quaternary ammonium salt thereof in admixture with pharmaceutically acceptable carriers, diluents or excipients.

4. A process for the preparation of a compound of formula (I) as defined in claim 1 which comprises
a) for the preparation of compounds of formula (I) wherein X is a group NR, reacting a compound of formula (II), 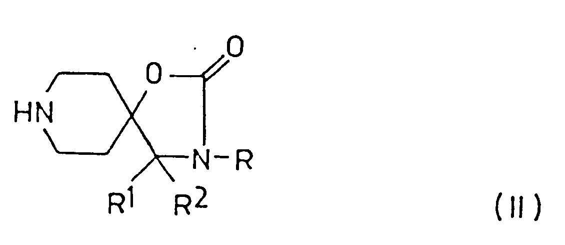 wherein R is as defined in claim 1 and R¹ and R² together are a methylene group, with a compound of formula (III), 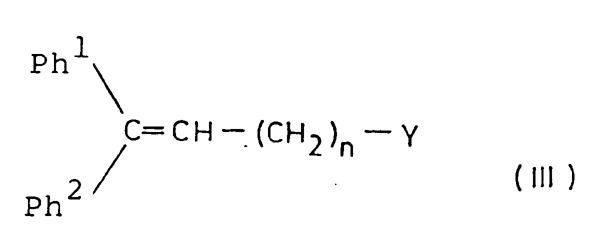 wherein Ph¹, Ph² and n are as defined in claim 1 and Y represents a halogen atom or a C₁₋₄alkylsulfonyloxy or arylsulfonyloxy group;
or
b) reacting a compound of formula (IV), 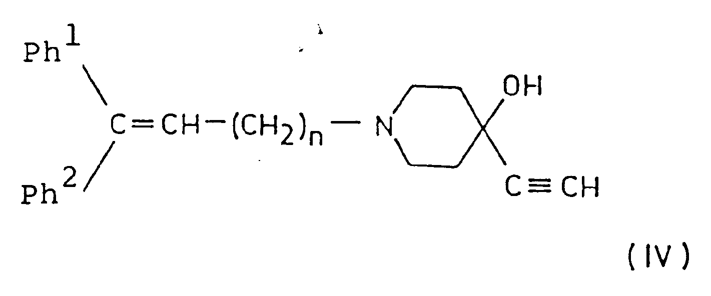 wherein Ph¹, Ph² and n are as defined above, and an isocyanate of formula R-NCO, wherein R is as defined above other than hydrogen,
followed by either
α) for the preparation of compounds of formula (I) wherein X is oxygen, cyclizing the resultant compound of formula (V), 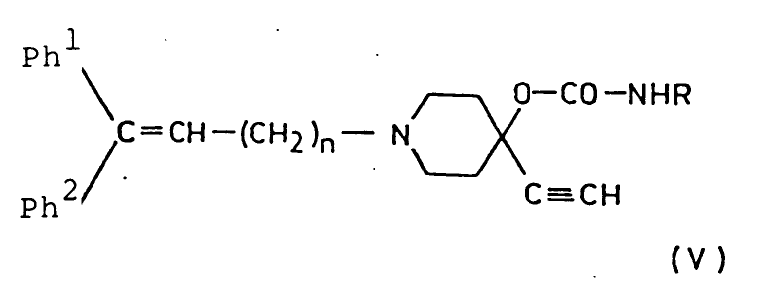 wherein R, Ph¹, Ph² and n are as defined above, in an acidic medium and reacting the resultant compound of formula (VI), 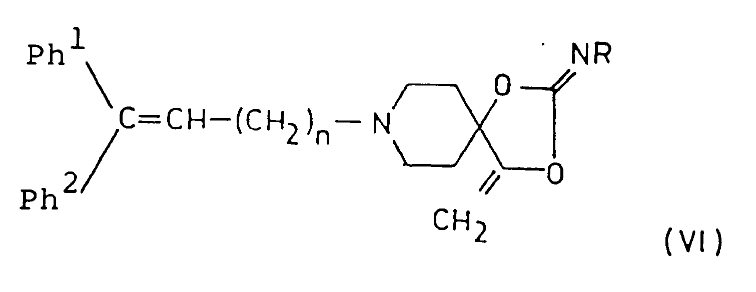 wherein R, Ph¹, Ph² and n are as defined above, obtained in a salt form, with water;
or
β) for the preparation of compounds of formula (I) wherein X is a group NR, cyclizing the thus-obtained compound of formula (V) as defined above, in a basic medium;
or
c) for the preparation of compounds of formula (I) wherein X is oxygen, cyclizing a compound of formula (V) as defined above in an acidic medium and reacting the resultant compound of formula (VI) as defined above, obtained in a salt form, with water,
or
d) for the preparation of compounds of formula (I) wherein X is a group NR, cyclizing the thus-obtained compound of formula (V) as defined above, in a basic medium;
or
e) for the preparation of compounds of formula (I) wherein X is a group NR, dehydrating a compound of formula (VII), 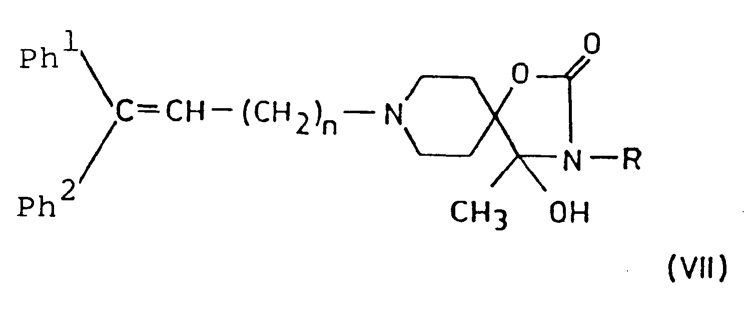 wherein R, Ph¹, Ph² and n are as defined above;
followed if desired or necessary,
by transforming a functional group of a thus-obtained compound of the formula (I), as defined above into another compound of formula (I) as defined in claim 1 in a known manner,
and/or
reacting a thus-obtained compound of the formula (I) as defined above with an acid to give an acid addition salt and/or reacting a salt of compound of the formula (I) as defined with a base to liberate the free basic form thereof and/or converting a thus-obtained compound of the formula (I) as defined above into its quaternary ammonium salt.

5. A process as claimed in claim 4 process a) wherein Y is either a chlorine or bromine atom or a methylsulfonyloxy or p-toluenesulfonyloxy group.

6. A process as claimed in claim 4, process a) or claim 5 which comprises carrying out the reaction in the presence of potassium iodide in a ketone-type solvent.

7. A process as claimed in claim 4, process b) wherein all the reaction steps are all carried out in one single step without isolating the compound of formula (V).

8. A process as claimed in claim 4 process c) which comprises carrying out the cyclization in dioxan in the presence of hydrogen chloride.

9. The use of a compound of formula (I) as defined in claim 1 in the manufacture of a medicament for the treatment of psychiatric disorders.

10. A process for the preparation of a pharmaceutical composition as defined in claim 3 which comprises admixing a compound of formula (I) as defined in claim 1 or a pharmaceutically acceptable acid addition or quaternary ammonium salt thereof with a pharmaceutically acceptable carrier, diluent or excipient.

## Claims (Claims for the following Contracting State(s): ES, GR)

1. A process for the preparation of a compound of formula (I) 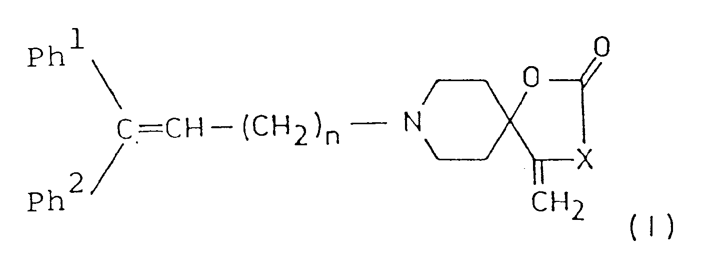 wherein
X represents an oxygen atom or a NR group, wherein
R represents a hydrogen atom or a C₁₋₁₂alkyl or C₃₋₆cycloalkyl group,
or a carbocyclic C₆₋₁₀aryl or carbocyclic C₆₋₁₀aryl-C₁₋₄alkyl group optionally substituted on the aromatic ring by one or more halogen atoms or C₁₋₄alkyl groups or C₁₋₄ al koxy groups.
Ph¹ and Ph², which may be the same or different, each represent a phenyl group unsubstituted or substituted by one or more halogen atoms or C₁₋₄alkyl, C₁₋₄alkoxy, trihalomethyl groups or hydroxyl groups optionally esterified by a C₁₋₄alkanoic acid; and
n is 1 or 2;
and optical isomers and mixtures thereof, and all solvates, hydrates, acid addition and quaternary ammonium salts thereof, which comprises
a) for the preparation of compounds of formula (I) wherein X is a group NR, reacting a compound of formula (II), 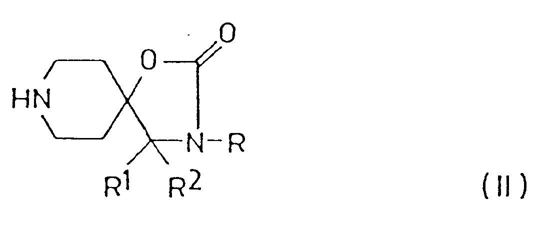 wherein R is as defined above and R¹ and R² together are a methylene group, with a compound of formula (III), 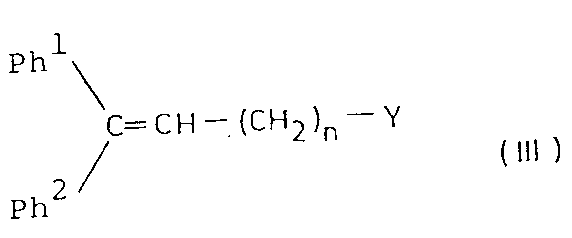 wherein Ph¹, Ph² and n are as defined above and Y represents a halogen atom or a C₁₋₄alkylsulfonyloxy or arylsulfonyloxy group;
or
b) reacting a compound of formula (IV), 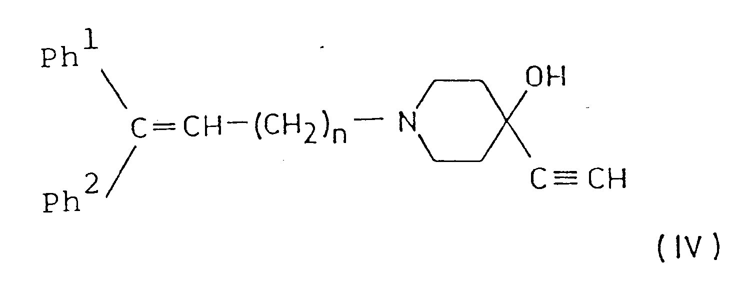 wherein Ph¹, ph² and n are as defined above, and an isocyanate of formula R-NCO, wherein R is as defined above other than hydrogen,
followed by either
α) for the preparation of compounds of formula (I) wherein X is oxygen, cyclizing the resultant compound of formula (V), 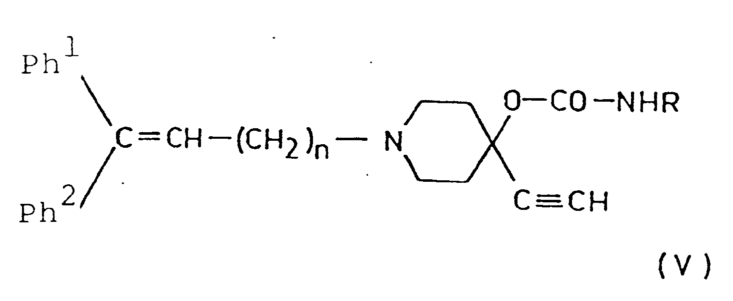 wherein R, Ph¹, Ph² and n are as defined above, in an acidic medium and reacting the resultant compound of formula (VI), 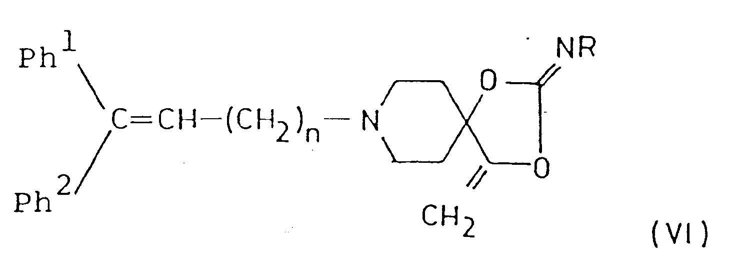 wherein R, Ph¹, Ph² and n are as defined above, obtained in a salt form, with water;
or
β) for the preparation of compounds of formula (I) wherein X is a group NR, cyclizing the thus-obtained compound of formula (V) as defined above, in a basic medium;
or
c) for the preparation of compounds of formula (I) wherein X is oxygen, cyclizing a compound of formula (V) as defined above in an acidic medium and reacting the resultant compound of formula (VI) as defined above, obtained in a salt form, with water
or
d) for the preparation of compounds of formula (I) wherein X is a group NR, cyclizing the thus-obtained compound of formula (V) as defined above, in a basic medium;
or
e) for the preparation of compounds of formula (I) wherein X is a group NR, dehydrating a compound of formula (VII), 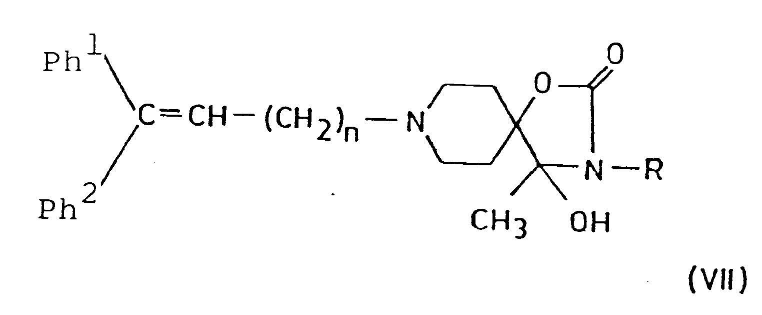 wherein R, Ph¹, Ph² and n are as defined above;
followed if desired or necessary,
by transforming a functional group of a thus-obtained compound of the formula (I), as defined above into another compound of formula (I) as defined above in a known manner,
and/or
reacting a thus-obtained compound of the formula (I) as defined above with an acid to give an acid addition salt and/or reacting a salt of compound of the formula (I) as defined with a base to liberate the free basic form thereof and/or converting a thus-obtained compound of the formula (I) as defined above into its quaternary ammonium salt.

2. A process as claimed in claim 1 process a) wherein Y is either a chlorine or bromine atom or a methylsulfonyloxy or p-toluenesulfonyloxy group.

3. A process as claimed in claim 1, process a) or claim 2 which comprises carrying out the reaction in the presence of potassium iodide in a ketone-type solvent.

4. A process as claimed in claim 1, process b) wherein all the reaction steps are all carried out in one single step without isolating the compound of formula (V).

5. A process as claimed in claim 1 process c) which comprises carrying out the cyclization in dioxan in the presence of hydrogen chloride.

6. A process as claimed in Claim 1 for the preparation of a compound as defined in claim 1 selected from the group comprising:
8-[4,4-bis(4-fluorophenyl)-3-butenyl]-4-methylene-2-oxo-1-oxa-3,8-diazaspiro[4,5]decane,
3-benzyl-8-[4,4-bis(4-fluorophenyl)-3-butenyl]-4-methylene-2-oxo-1-oxa-3,8-diazaspiro[4,5]decane,
8-[4,4-bis(4-fluorophenyl)-3-butenyl]-4-methylene-2-oxo-1,3-dioxa-8-azaspiro[4,5]decane and
8-[4,4-bis(4-fluorophenyl)-3-butenyl]-3-methyl-4-methylene-2-oxo-1-oxa-3,8-diazaspiro[4,5]decane
and optical isomers and mixtures thereof, and all solvates, hydrates, acid addition and quaternary ammonium salts thereof.

7. The use of a compound of formula (I) as defined in claim 1 in the manufacture of a medicament for the treatment of psychiatric disorders.

8. A process for the preparation of a pharmaceutical composition which comprises as active ingredient a compound of formula (I) as defined in claim 1 or a pharmaceutically acceptable acid addition or quaternary ammonium salt thereof, wherein the active ingredient is admixed with a pharmaceutically acceptable carrier, diluent or excipient.

## Patentansprüche (Patentansprüche für folgende(n) Vertragsstaat(en): AT, BE, CH, DE, DK, FR, GB, IT, LI, LU, NL, SE)

1. Verbindung der Formel (I) 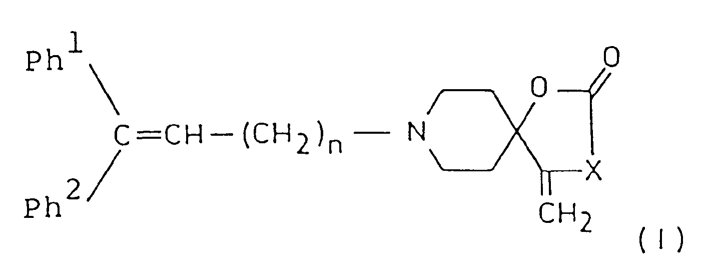 worin
X ein Sauerstoffatom oder eine NR-Gruppe darstellt, wobei
R ein Wasserstoffatom oder eine C₁₋₁₂-Alkyl oder C₃₋₆-Cycloalkylgruppe, oder eine carbocyclische C₆₋₁₀-Aryl- oder carbocyclische C₆₋₁₀-Aryl-C₁₋₄-alkylgruppe darstellt, die am aromatischen Ring wahlweise mit einem oder mehreren Halogenatomen, C₁₋₄-Alkylgruppen oder C₁₋₄-Alkoxygruppen substituiert sind,
Ph¹ und Ph², welche gleich oder verschieden sein können, jeweils eine Phenylgruppe darstellen, die unsubstituiert oder mit einem oder mehreren Halogenatomen oder C₁₋₄-Alkyl-, C₁₋₄-Alkoxy-, Trihalomethylgruppen oder Hydroxylgruppen, die wahlweise mit einer C₁₋₄-Alkansäure verestert sind, substituiert ist; und
n 1 oder 2 ist;
und deren optische Isomere und Mischungen und alle ihre Solvate, Hydrate, Säureadditions- und quaternären Ammoniumsalze.

2. Verbindung gemäß Anspruch 1, ausgewählt aus der Gruppe, welche umfaßt:
8-[4,4-Bis(4-fluorphenyl)-3-butenyl]-4-methylen-2-oxo-1-oxa-3,8-diazaspiro[4,5]decan,
3-Benzyl-8-[4,4-bis(4-fluorphenyl)-3-butenyl]-4-methylen-2-oxo-1-oxa-3,8-diazaspiro[4,5]decan,
8-[4,4-Bis(4-fluorphenyl)-3-butenyl]-4-methylen-2-oxo-1,3-dioxa-8-azaspiro[4,5]decan und
8-[4,4-Bis(4-fluorphenyl)-3-butenyl]-3-methyl-4-methylen-2-oxo-1-oxa-3,8-diazaspiro[4,5]decan
und ihren optischen Isomeren und Mischungen und allen ihren Solvaten, Hydraten, säureadditions- und quaternären Ammoniumsalzen.

3. Pharmazeutische Zusammensetzung, die als Wirkstoff eine Verbindung der Formel (I) gemäß Anspruch 1 oder eines ihrer pharmazeutisch annehmbaren Säureadditions- oder quaternären Ammoniumsalze in Mischung mit pharmazeutisch annehmbaren Trägern, verdünnungsmittel oder Korrigenzien umfaßt.

4. Verfahren zur Herstellung einer Verbindung der Formel (I) gemäß Anspruch 1, umfassend:
a) zur Herstellung von Verbindungen der Formel (I), in denen X eine NR-Gruppe ist, Umsetzung einer Verbindung der Formel (II) 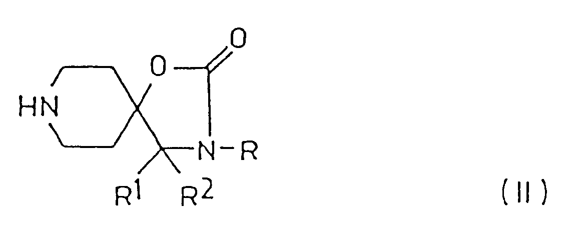 worin R wie in Anspruch 1 definiert ist und R¹ und R² zusammen eine Methylengruppe sind, mit einer Verbindung der Formel (III) 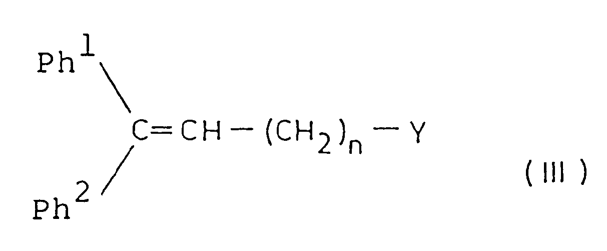 worin Ph¹, Ph² und n wie in Anspruch 1 definiert sind und Y ein Halogenatom oder eine C₁₋₄-Alkylsulfonyloxy- oder Arylsulfonyloxygruppe darstellt;
oder
b) Umsetzung einer Verbindung der Formel (IV) 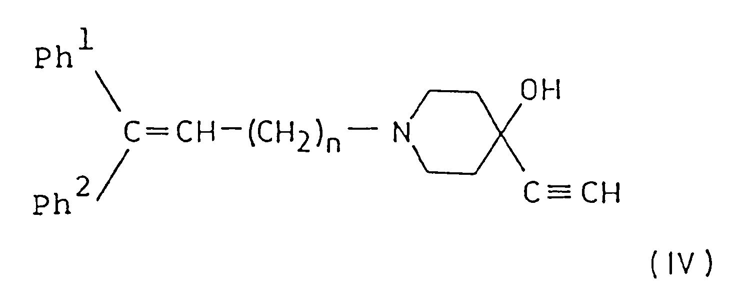 worin Ph¹, Ph² und n wie oben definiert sind, und eines Isocyanats der Formel R-NCO, worin R mit Ausnahme von Wasserstoff wie oben definiert ist,
gefolgt von entweder
α) zur Herstellung von Verbindungen der Formel (I), in denen X Sauer- stoff ist, Cyclisierung der resultierenden Verbindung der Formel V 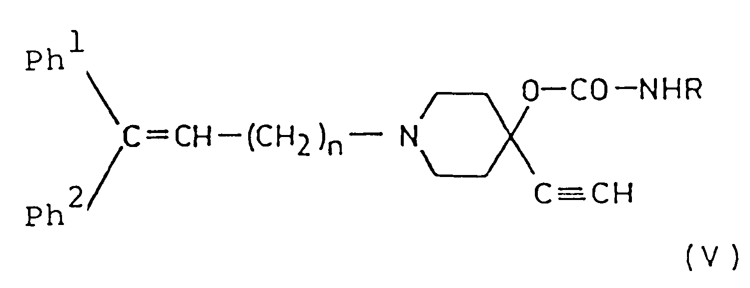 worin R, Ph¹, Ph² und n wie oben definiert sind, in einem sauren Medium und Umsetzung der resultierenden Verbindung der Formel (VI) 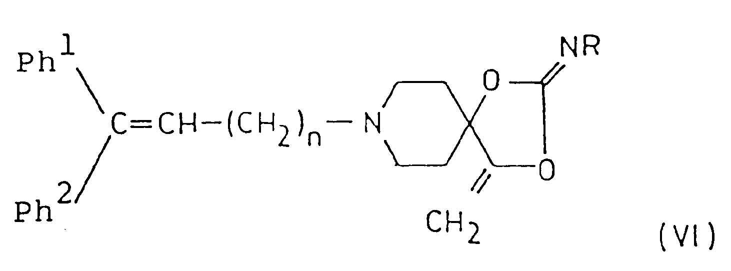 worin R, Ph¹, Ph² und n wie oben definiert sind, die in Salzform erhalten wird, mit Wasser;
oder
β) zur Herstellung von Verbindungen der Formel (I), in denen X eine NR-Gruppe ist, Cyclisierung der so erhaltenen Verbindung der wie oben definierten Formel (V) in einem basischen Medium; oder
c) zur Herstellung von Verbindungen der Formel (I), in denen X Sauerstoff ist, Cyclisierung einer Verbindung der wie oben definierten Formel (V) in einem sauren Medium und Umsetzung der resultierenden Verbindung der wie oben definierten Formel (VI), die in Salzform erhalten wird, mit Wasser;
oder
d) zur Herstellung von Verbindungen der Formel (I), in denen X eine NR-Gruppe ist, Cyclisierung der so erhaltenen Verbindung der wie oben definierten Formel (V) in einem basischen Medium; oder
e) zur Herstellung von Verbindungen der Formel (I), in denen X eine NR-Gruppe ist, Dehydratisierung einer Verbindung der Formel (VII) 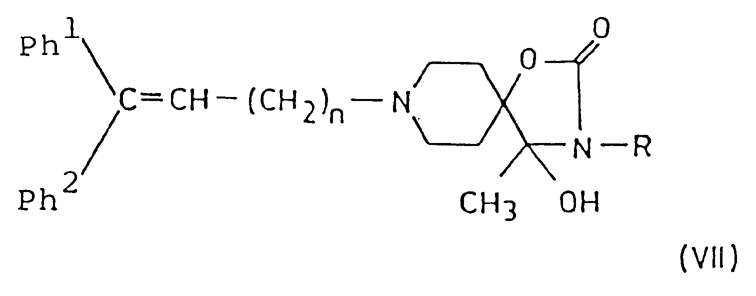 worin R, Ph¹, Ph² und n wie oben definiert sind;
gefolgt falls gewünscht oder erforderlich,
von Umwandlung einer funktionellen Gruppe einer so erhaltenen Verbindung der wie oben definierten Formel (I) in eine andere Verbindung der wie in Anspruch 1 definierten Formel (I) auf bekannte Weise,
und/oder
Umsetzung einer so erhaltenen Verbindung der wie oben definierten Formel (I) mit einer Säure zur Bildung eines Säureadditionssalzes und/oder Umsetzung eines Salzes einer Verbindung der wie oben definierten Formel (I) mit einer Base zur Freisetzung ihrer freien Base-Form und/oder Umwandlung einer so erhaltenen Verbindung der wie oben definierten Formel (I) in ihr quaternäres Ammoniumsalz.

5. Verfahren gemäß Anspruch 4, Verfahren a), dadurch **gekennzeichnet,** daß Y entweder ein Chlor- oder ein Bromatom oder eine Methylsulfonyloxy- oder p-Toluolsulfonyloxygruppe ist.

6. Verfahren gemäß Anspruch 4, Verfahren a) oder Anspruch 5, das die Ausführung der Reaktion in Gegenwart von Kaliumjodid in einem Ketonlösungsmittel umfaßt.

7. Verfahren gemäß Anspruch 4, Verfahren b), dadurch **gekennzeichnet,** daß alle Reaktionsschritte in einem einzigen Schritt ohne Isolierung der Verbindung der Formel (V) ausgeführt werden.

8. Verfahren gemäß Anspruch 4, Verfahren c), umfassend die Cyclisierung in Dioxan in Gegenwart von Chlorwasserstoff.

9. Verwendung einer Verbindung der wie in Anspruch 1 definierten Formel (I) bei der Herstellung eines Medikaments zur Behandlung von psychiatrischen Störungen.

10. Verfahren zur Herstellung einer pharmazeutischen Zusammensetzung gemäß Anspruch 3, umfassend das Mischen einer Verbindung der wie in Anspruch 1 definierten Formel (I) oder eines pharmazeutisch annehmbaren Säureadditions- oder quaternären Ammoniumsalzes davon mit einem pharmazeutisch annehmbaren Träger, Verdünnungsmittel oder Korrigens.

## Patentansprüche (Patentansprüche für folgende(n) Vertragsstaat(en): ES, GR)

1. Verfahren zur Herstellung einer Verbindung der Formel (I) 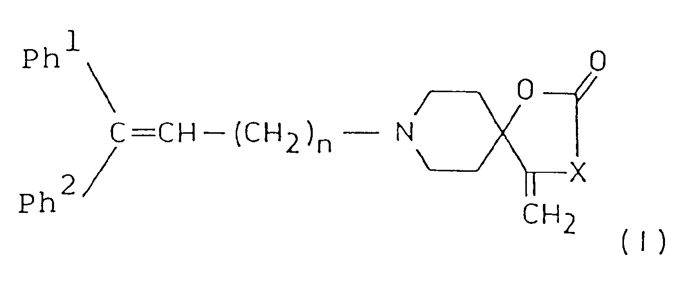 worin
X ein Sauerstoffatom oder eine NR-Gruppe darstellt, wobei
R ein Wasserstoffatom oder eine C₁₋₁₂-Alkyl- oder C₃₋₆-Cycloalkylgruppe, oder eine carbocyclische C₆₋₁₀-Aryl- oder carbocyclische C₆₋₁₀-Aryl-C₁₋₄-alkyl-Gruppe darstellt, die wahlweise am aromatischen Ring mit einem oder mehreren Halogenatomen oder C₁₋₄-Alkylgruppen oder C₁₋₄-Alkoxygruppen substituiert sind,
Ph¹ und Ph², welche gleich oder verschieden sein können, jeweils eine Phenylgruppe darstellen, die unsubstituiert oder substituiert mit einem oder mehreren Halogenatomen oder C₁₋₄-Alkyl-, C₁₋₄-Alkoxy-, Trihalomethylgruppen oder Hydroxylgruppen, die wahlweise mit einer C₁₋₄-Alkansäure verestert sind, ist; und
n 1 oder 2 ist;
und deren optischen Isomeren und Mischungen und allen ihren Solvaten, Hydraten, Säureadditions- und quaternären Ammoniumsalzen, umfassend:
a) zur Herstellung von Verbindungen der Formel (I), in denen X eine NR-Gruppe ist, Umsetzung einer Verbindung der Formel (II) 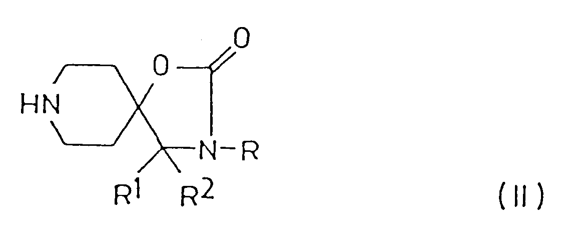 worin R wie oben definiert ist und R¹ und R² zusammen eine Methylengruppe sind, mit einer Verbindung der Formel (III) 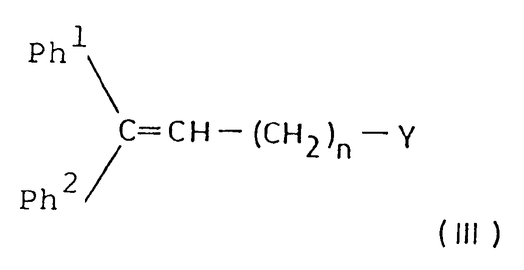 worin Ph¹, Ph² und n wie oben definiert sind und Y ein Halogenatom oder eine C₁₋₄-Alkylsulfonyloxy- oder Arylsulfonyloxygruppe darstellt;
oder
b) Umsetzung einer Verbindung der Formel (IV) 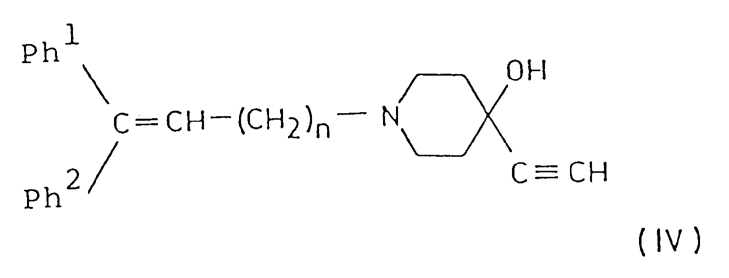 worin Ph¹, Ph² und n wie oben definiert sind, und einem Isocyanat der Formel R-NCO, worin R mit Ausnahme von Wasserstoff wie oben definiert ist, gefolgt von entweder
α) zur Herstellung von Verbindungen der Formel (I), in denen X Sauerstoff ist, Cyclisierung der resultierenden Verbindung der Formel (V) 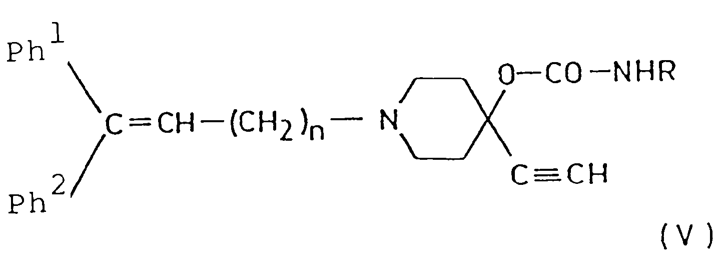 worin R, Ph¹, Ph² und n wie oben definiert sind, in einem sauren Medium und Umsetzung der resultierenden Verbindung der Formel (VI) 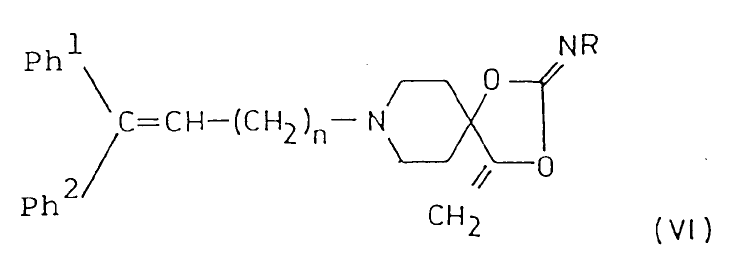 worin R, Ph¹, Ph² und n wie oben definiert sind, das in einer Salzform erhalten wurde, mit Wasser;
oder
β) zur Herstellung von Verbindungen der Formel (I), in denen X eine NR-Gruppe ist, Cyclisierung der so erhaltenen Verbindung der wie oben definierten Formel (V) in einem basischen Medium; oder
c) zur Herstellung von Verbindungen der Formel (I), in denen X Sauerstoff ist, Cyclisierung einer Verbindung der wie oben definierten Formel (V) in einem sauren Medium und Umsetzung der resultierenden Verbindung der wie oben definierten Formel (VI), die in Salzform erhalten wurde, mit Wasser;
oder
d) zur Herstellung von Verbindungen der Formel (I), in denen X eine NR-Gruppe ist, Cyclisierung der so erhaltenen Verbindung der wie oben definierten Formel (V) in einem basischen Medium; oder
e) zur Herstellung von Verbindungen der Formel (I), in denen X eine NR-Gruppe ist, Dehydratisierung einer Verbindung der Formel (VII) 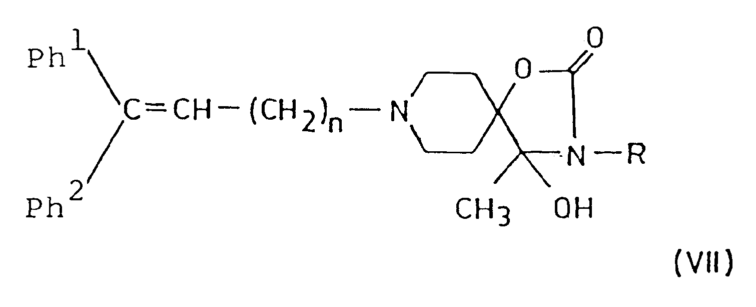 worin R, Ph¹, Ph² und n wie oben definiert sind;
gefolgt falls gewünscht oder erforderlich,
von Umwandlung einer funktionellen Gruppe einer so erhaltenen Verbindung der Formel (I), wie oben definiert, in eine andere Verbindung der wie oben definierten Formel (I) auf bekannte Weise,
und/oder
Umsetzung einer so erhaltenen Verbindung der wie oben definierten Formel (I) mit einer Säure unter Bildung eines Säureadditionssalzes und/oder Umsetzung eines Salzes einer Verbindung der wie oben definierten Formel (I) mit einer Base zur Freisetzung ihrer freien Base-Form und/oder Umwandlung einer so erhaltenen Verbindung der wie oben definierten Formel (I) in ihr quaternäres Ammoniumsalz.

2. Verfahren gemäß Anspruch 1 Verfahren a), dadurch **gekennzeichnet,** daß Y entweder ein Chlor- oder Bromatom oder eine Methylsulfonyloxy- oder p-Toluolsulfonyloxygruppe ist.

3. Verfahren gemäß Anspruch 1 Verfahren a) oder Anspruch 2, umfassend die Ausführung der Reaktion in Gegenwart von Kaliumjodid in einem Keton-Lösungsmittel.

4. Verfahren gemäß Anspruch 1, Verfahren b), dadurch **gekennzeichnet,** daß alle Reaktionsschritte in einem einzigen Schritt ohne Isolierung der Verbindung der Formel (V) ausgeführt werden.

5. Verfahren gemäß Anspruch 1, Verfahren c), umfassend die Ausführung der Cyclisierung in Dioxan in Gegenwart von Chlorwasserstoff.

6. Verfahren gemäß Anspruch 1 zur Herstellung einer Verbindung gemäß Anspruch 1, ausgewählt aus der Gruppe, welche umfaßt:
8-[4,4-Bis(4-fluorphenyl)-3-butenyl]-4-methylen-2-oxo-1-oxa-3,8-diazaspiro[4,5]decan,
3-Benzyl-8-[4,4-bis(4-fluorphenyl)-3-butenyl]-4-methylen-2-oxo-1-oxa-3,8-diazaspiro[4,5]decan,
8-[4,4-Bis(4-fluorphenyl)-3-butenyl]-4-methylen-2-oxo-1,3-dioxa-8-azaspiro[4,5]decan und
8-[4,4-Bis(4-fluorphenyl)-3-butenyl]-3-methyl-4-methylen-2-oxo-1-oxa-3,8-diazaspiro[4,5]decan
und ihren optischen Isomeren und Mischungen und allen ihren Solvaten, Hydraten, Säureadditions- und quaternären Ammoniumsalzen.

7. Verwendung einer Verbindung der Formel (I) gemäß Anspruch 1 bei der Herstellung eines Medikaments zur Behandlung von psychiatrischen Störungen.

8. Verfahren zur Herstellung einer pharmazeutischen Zusammensetzung, die als Wirkstoff eine Verbindung der wie in Anspruch 1 definierten Formel (I) oder eines ihrer pharmazeutisch annehmbaren Säureadditions- oder quaternären Ammoniumsalze umfaßt, dadurch **gekennzeichnet,** daß der Wirkstoff mit einem pharmazeutisch annehmbaren Träger, Verdünnungsmittel oder Korrigens gemischt wird.

## Revendications (Revendications pour l'(les) Etat(s) contractant(s) suivant(s): AT, BE, CH, DE, DK, FR, GB, IT, LI, LU, NL, SE)

1. Composé de formule (I), 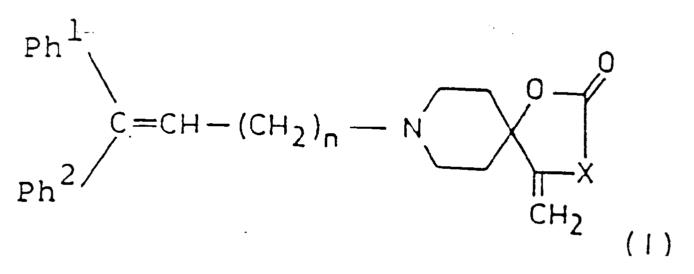 dans laquelle
X représente un atome d'oxygène ou un groupe NR, dans lequel
R représente un atome d'hydrogène ou un groupe alkyle en C₁-C₁₂ ou cycloalkyle en C₃-C₆, ou un groupe aryle en C₆-C₁₀ carbocyclique ou un aryl(en C₆-C₁₀)alkyle(en C₁-C₄) carbocyclique éventuellement substitué, sur le noyau aromatique, par un ou plusieurs atomes d'halogène, groupes alkyle en C₁-C₄ ou groupes alcoxy en C₁-C₄;
Ph¹ et Ph², qui peuvent être identiques ou différents, représentent chacun un groupe phényle non substitué ou substitué par un ou plusieurs atomes d'halogène ou groupes alkyle en C₁-C₄, alcoxy en C₁-C₄, trihalogénométhyle ou hydroxyle, éventuellement estérifiés par un acide alcanoïque en C₁-C₄; et
n est égal à 1 ou 2;
et leurs isomères optiques et leurs mélanges, et tous leurs solvates, hydrates, sels d'addition avec un acide et sels d'ammonium quaternaire.

2. Composé selon la revendication 1, choisi dans le groupe comprenant :
le 8-[4,4-bis(4-fluorophenyl)-3-butényl]-4-méthylène-2-oxo-1-oxa-3,8-diazaspiro[4,5]décane,
le 3-benzyl-8-[4,4-bis(4-fluorophényl)-3-butényl]-4-methylène-2-oxo-1-oxa-3,8-diazaspiro[4,5]décane,
le 8-[4,4-bis(4-fluorophényl)-3-butényl]-4-méthylène-2-oxo-1,3-dioxa-8-azaspiro[4,5]décane et
le 8-[4,4-bis(4-fluorophényl)-3-butényl]-3-méthyl-4-méthylène-2-oxo-1-oxa-3,8-diazaspiro[4,5]décane,
et leurs isomères optiques et leurs mélanges, et tous leurs solvates, hydrates, sels d'addition avec un acide et sels d'ammonium quaternaire.

3. Composition pharmaceutique qui comprend, comme ingrédient actif, un composé de formule (I) définie dans la revendication 1 ou un de ses sels pharmaceutiquement acceptables d'addition avec un acide ou d'ammonium quaternaire, en mélange avec des véhicules, diluants ou excipients pharmaceutiquement acceptables.

4. Procédé de préparation d'un composé de formule (I) définie dans la revendication 1, qui comprend
a) pour la préparation de composés de formule (I), dans laquelle X est un groupe NR, la réaction d'un composé de formule (II) 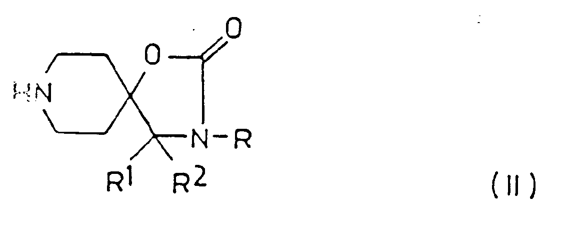 dans laquelle R est tel que défini dans la revendication 1 et R¹ et R² constituent ensemble un groupe méthylène, avec un composé de formule (III), 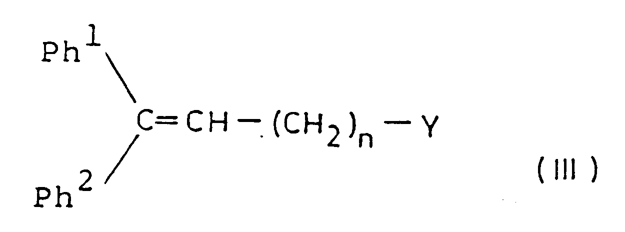 dans laquelle Ph¹, Ph² et n sont tels que définis dans la revendication 1, et Y représente un atome d'halogène ou un groupe alkyl(en C₁-C₄)sulfonyloxy ou arylsulfonyloxy;
ou
b) la réaction d'un composé de formule (IV) 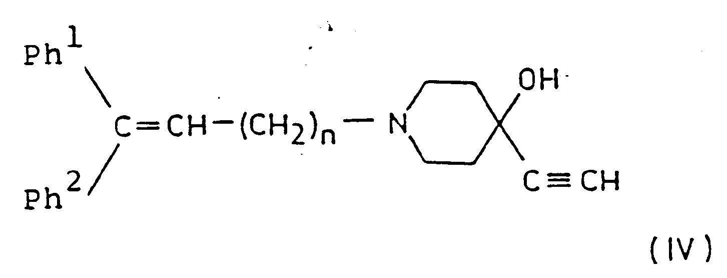 dans laquelle Ph¹, Ph² et n sont tels que définis ci-dessus, et d'un isocyanate de formule R-NCO, dans laquelle R est tel que défini ci-dessus, différent toutefois d'un hydrogène, suivie soit
α) pour la préparation de composés de formule (I), dans laquelle X est un atome d'oxygène, de la cyclisation du composé résultant de formule (V), 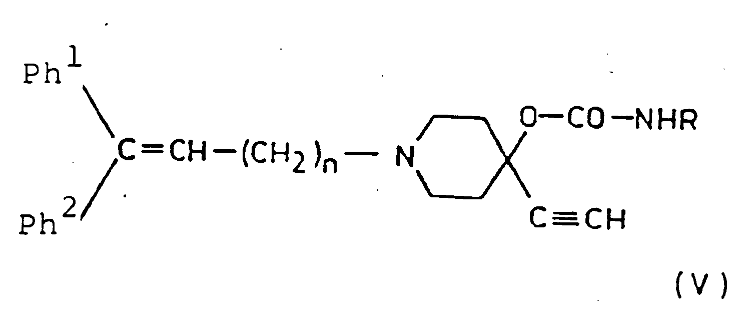 dans laquelle R, Ph¹, Ph² et n sont tels que définis ci-dessus, dans un milieu acide, et de la réaction du composé résultant de formule (VI) 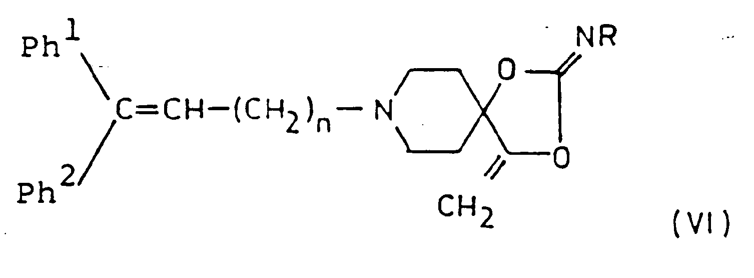 dans laquelle R, Ph¹, Ph² et n sont tels que définis ci-dessus, obtenus sous la forme d'un sel, avec de l'eau; soit
β) pour la préparation de composés de formule (I), dans laquelle X est un groupe NR, la cyclisation du composé ainsi obtenu de formule (V) définie ci-dessus, dans un milieu basique;
ou
c) pour la préparation de composés de formule (I), dans laquelle X est un atome d'oxygène, la cyclisation d'un composé de formule (V) définie ci-dessus, dans un milieu acide, et la réaction du composé de formule (VI) résultant, tel que défini ci-dessus, obtenu sous la forme d'un sel, avec de l'eau;
ou
d) pour la préparation de composés de formule (I), dans laquelle X est un groupe NR, la cyclisation du composé ainsi obtenu de formule (V) définie ci-dessus, dans un milieu basique;
ou
e) pour la préparation de composés de formule (I), dans laquelle X est un groupe NR, la déshydratation d'un composé de formule (VII) 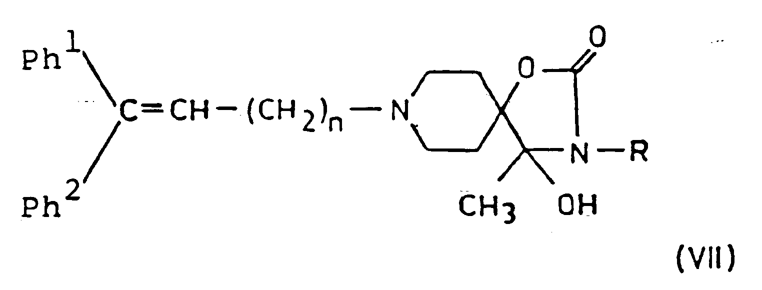 dans laquelle R, Ph¹, Ph² et n sont tels que définis ci-dessus;
suivie, le cas échéant, de
la transformation d'un groupe fonctionnel d'un composé ainsi obtenu de formule (I) définie ci-dessus en un autre composé de formule (I) définie dans la revendication 1, d'une manière connue,
et/ou
la réaction d'un composé ainsi obtenu de formule (I) définie ci-dessus avec un acide, pour donner un sel d'addition avec un acide, et/ou la réaction d'un sel d'un composé de formule (I) définie ci-dessus avec une base, pour libérer sa forme de base libre, et/ou la transformation d'un composé ainsi obtenu de formule (I) définie ci-dessus en son sel d'ammonium quaternaire.

5. Procédé selon la revendication 4, procédé a), dans lequel Y est un atome de chlore ou de brome, ou bien un groupe méthylsulfonyloxy ou p-toluènesulfonyloxy.

6. Procédé selon la revendication 4, procédé a), ou selon la revendication 5, qui comprend la mise en oeuvre de la réaction en présence d'iodure de potassium dans un solvant cétonique.

7. Procédé selon la revendication 4, procédé b), dans lequel toutes les étapes réactionnelles sont réalisées en une seule étape, sans isolement du composé de formule (V).

8. Procédé selon la revendication 4, procédé c), qui comprend la mise en oeuvre de la cyclisation dans le dioxane, en présence de chlorure d'hydrogène.

9. Utilisation d'un composé de formule (I) définie dans la revendication 1 dans la fabrication d'un médicament pour le traitement de troubles psychiatriques.

10. Procédé de préparation d'une composition pharmaceutique selon la revendication 3, qui comprend le mélange d'un composé de formule (I) définie dans la revendication 1, ou d'un de ses sels d'addition avec un acide ou sels d'ammonium quaternaire pharmaceutiquement acceptables avec un véhicule, diluant ou excipient pharmaceutiquement acceptable.

## Revendications (Revendications pour l'(les) Etat(s) contractant(s) suivant(s): ES, GR)

1. Procédé de préparation d'un composé de formule (I) 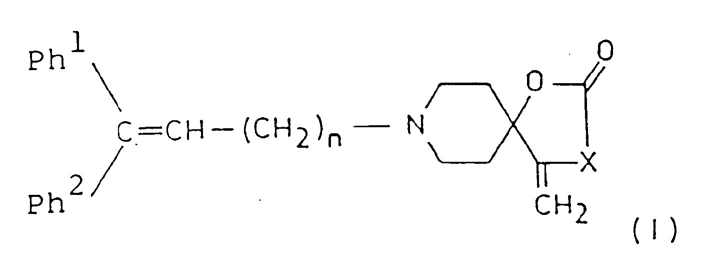 dans laquelle
X représente un atome d'oxygène ou un groupe NR, dans lequel
R représente un atome d'hydrogène ou un groupe alkyle en C₁-C₁₂ ou cycloalkyle en C₃-C₆, ou un groupe aryle en C₆-C₁₀ carbocyclique ou un aryl(en C₆-C₁₀)alkyle(en C₁-C₄) carbocyclique éventuellement substitué, sur le noyau aromatique, par un ou plusieurs atomes d'halogène, groupes alkyle en C₁-C₄ ou groupes alcoxy en C₁-C₄;
Ph¹ et Ph², qui peuvent être identiques ou différents, représentent chacun un groupe phényle non substitué ou substitué par un ou plusieurs atomes d'halogène ou groupes alkyle en C₁-C₄, alcoxy en C₁-C₄, trihalogénométhyle ou hydroxyle, éventuellement estérifiés par un acide alcanoïque en C₁-C₄; et
n est égal à 1 ou 2;
et de leurs isomères optiques et de leurs mélanges, et de tous leurs solvates, hydrates, sels d'addition avec un acide et sels d'ammonium quaternaire, procédé qui comprend :
a) pour la préparation de composés de formule (I), dans laquelle X est un groupe NR, la réaction d'un composé de formule (II) 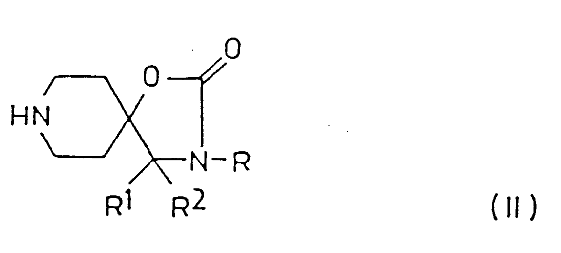 dans laquelle R est tel que défini ci-dessus et R¹ et R² constituent ensemble un groupe méthylène, avec un composé de formule (III), 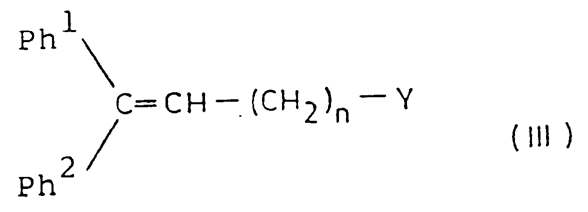 dans laquelle Ph¹, Ph² et n sont tels que définis ci-dessus, et Y représente un atome d'halogène ou un groupe alkyl(en C₁-C₄)sulfonyloxy ou arylsulfonyloxy;
ou
b) la réaction d'un composé de formule (IV) 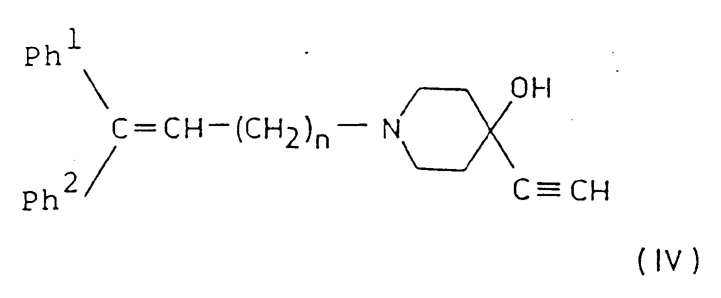 dans laquelle Ph¹, Ph² et n sont tels que définis ci-dessus, et d'un isocyanate de formule R-NCO, dans laquelle R est tel que défini ci-dessus, différent toutefois d'un hydrogène, suivie soit
α) pour la préparation de composés de formule (I), dans laquelle X est un atome d'oxygène, de la cyclisation du composé résultant de formule (V), 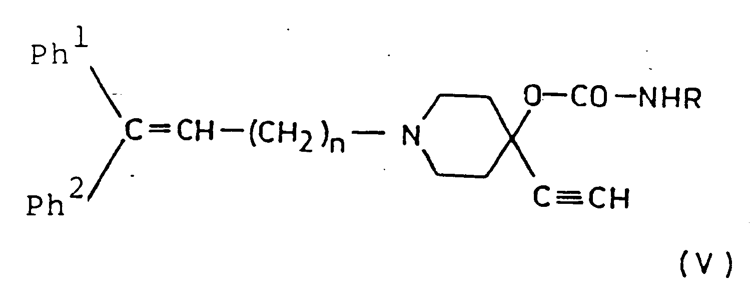 dans laquelle R, Ph¹, Ph² et n sont tels que définis ci-dessus, dans un milieu acide, et de la réaction du composé résultant de formule (VI) 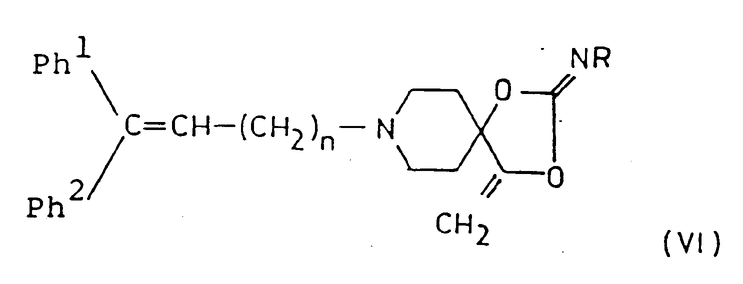 dans laquelle R, Ph¹, Ph² et n sont tels que définis ci-dessus, obtenus sous la forme d'un sel, avec de l'eau;
soit
β) pour la préparation de composés de formule (I), dans laquelle X est un groupe NR, la cyclisation du composé ainsi obtenu de formule (V) définie ci-dessus, dans un milieu basique;
ou
c) pour la préparation de composés de formule (I), dans laquelle X est un atome d'oxygène, la cyclisation d'un composé de formule (V) définie ci-dessus, dans un milieu acide, et la réaction du composé de formule (VI) résultant, tel que défini ci-dessus, obtenu sous la forme d'un sel, avec de l'eau;
ou
d) pour la préparation de composés de formule (I), dans laquelle X est un groupe NR, la cyclisation du composé ainsi obtenu de formule (V) définie ci-dessus, dans un milieu basique;
ou
e) pour la préparation de composés de formule (I), dans laquelle X est un groupe NR, la déshydratation d'un composé de formule (VII) 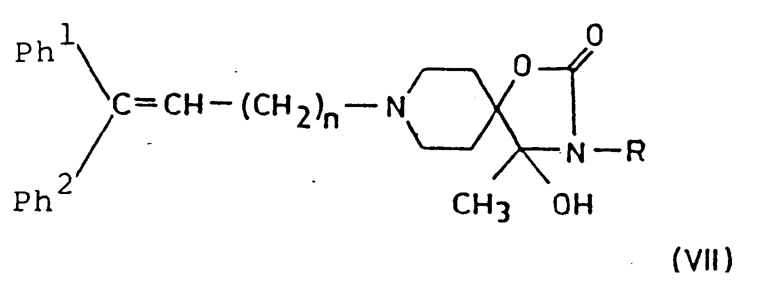 dans laquelle R, Ph¹, Ph² et n sont tels que définis ci-dessus;
suivie, le cas échéant, de
la transformation d'un groupe fonctionnel d'un composé ainsi obtenu de formule (I) définie ci-dessus en un autre composé de formule (I) définie ci-dessus, d'une manière connue,
et/ou
la réaction d'un composé ainsi obtenu de formule (I) définie ci-dessus avec un acide, pour donner un sel d'addition avec un acide, et/ou la réaction d'un sel d'un composé de formule (I) définie ci-dessus avec une base, pour libérer sa forme de base libre, et/ou la transformation d'un composé ainsi obtenu de formule (I) définie ci-dessus en son sel d'ammonium quaternaire.

2. Procédé selon la revendication 1, procédé a), dans lequel Y est un atome de chlore ou de brome, ou bien un groupe méthylsulfonyloxy ou p-toluènesulfonyloxy.

3. Procédé selon la revendication 1, procédé a), ou selon la revendication 2, qui comprend mise en oeuvre de la réaction en présence d'iodure de potassium dans un solvant cétonique.

4. Procédé selon la revendication 1, procédé b), dans lequel toutes les étapes réactionnelles sont réalisées en une seule etape, sans isolement du composé de formule (V).

5. Procédé selon la revendication 1, procédé c), qui comprend la mise en oeuvre de la cyclisation dans le dioxane, en présence de chlorure d'hydrogène.

6. Procédé selon la revendication 1, pour la préparation d'un composé selon la revendication 1, choisi dans le groupe comprenant :
le 8-[4,4-bis(4-fluorophényl)-3-butényl]-4-méthylène-2-oxo-1-oxa-3,8-diazaspiro[4,5]décane,
le 3-benzyl-8-[4,4-bis(4-fluorophényl)-3-butényl]-4-méthylène-2-oxo-1-oxa-3,8-diazaspiro[4,5]décane,
le 8-[4,4-bis(4-fluorophényl)-3-butényl]-4-méthylène-2-oxo-1,3-dioxa-8-azaspiro[4,5]décane et
le 8-[4,4-bis(4-fluorophényl)-3-butényl]-3-méthyl-4-méthylène-2-oxo-1-oxa-3,8-diazaspiro[4,5]décane,
et leurs isomères optiques et leurs mélanges, et tous leurs solvates, hydrates, sels d'addition avec un acide et sels d'ammonium quaternaire.

7. Utilisation d'un composé de formule (I) définie dans la revendication 1 dans la fabrication d'un médicament pour le traitement de troubles psychiatriques.

8. Procédé de préparation d'une composition pharmaceutique qui comprend, comme substance active, un composé de formule (I) définie dans la revendication 1, ou un de ses sels d'addition avec un acide ou sels d'ammonium quaternaire pharmaceutiquement acceptables, dans lequel la substance active est mélangée avec un véhicule, diluant ou excipient pharmaceutiquement acceptable.
